# EUROPEAN PATENT APPLICATION

(11) **EP 1 953 226 A1**
(43) Date of publication of application: **06.08.2008**
(21) Application number: 06832572.9
(22) Date of filing: 14.11.2006
(51) Int. Cl.: C12N 15/09, C07K 14/47, C07K 16/18, C12N 1/15, C12N 1/19, C12N 1/21, C12N 5/10, G01N 33/53, C12P 21/08

(54) **MEMBRANE MOLECULE EXPRESSED SPECIFICALLY IN ACTIVATED PLASMACYTOID DENDRITIC CELL**

(30) Priority: 14.11.2005 JP 2005329432
(71) Applicant: Riken, Wako-shi, Saitama 351-0198 (JP)
(72) Inventor: WATARAI, Hiroshi, Yokohama-shi Kanagawa 230-0045 (JP); SEKINE, Etsuko, Yokohama-shi Kanagawa 230-0045 (JP); TANIGUCHI, Masaru, Yokohama-shi Kanagawa 230-0045 (JP)
(74) Representative: Polypatent
(86) International application number: PCT/JP2006/322610
(87) International publication number: WO 2007/055369

(57) **Abstract**

It is an object of the present invention to identify a membrane molecule that is specifically expressed on activated plasmacytoid dendritic cells, for the purpose of preventing or improving affection or disease such as cancer, autoimmune disease, allergy, or infectious disease by regulating the functions of dendritic cells capable of integrating an immune system. The present invention provides a protein having any one of the following amino acid sequences:
(a) an amino acid sequence as shown in SEQ ID NO: 2;
(b) an amino acid sequence, which comprises a deletion, substitution, insertion and/or addition of one or several amino acid residues with respect to the amino acid sequence as shown in SEQ ID NO: 2, and which is capable of regulating the functions of dendritic cells; and
(c) an amino acid sequence, which has homology of 80% or more with the amino acid sequence as shown in SEQ ID NO: 2, and which is capable of regulating the functions of dendritic cells.

## Description

### TECHNICAL FIELD

The present invention relates to a membrane molecule which is expressed on activated plasmacytoid dendritic cells (PDC), DNA encoding the membrane molecule, an antibody against the membrane molecule, and a method of separating and detecting plasmacytoid dendritic cells using the antibody.

### BACKGROUND ART

It has been known that dendritic cells (DC) are differentiated and get mature from CD34 positive cells acting as precursor cells existing in the bone marrow of a living body, and that the dendritic cells play an important role as antigen presenting cells (APC) in induction, maintenance, expansion and control of immune response. DC that acts as professional APC is activated by stimulation of a natural immune response, and it induces an antigen-specific acquired immune response. In addition, DC generates various types of cytokines by itself, so that it plays an important role also in a natural immune response during an infectious episode. Expression of each TLR in DC is clearly different among MoDC, myeloid DC (MDC) in peripheral blood, and plasmacytoid DC (PDC). It has also been revealed that expression of a cytokine or chemokine receptor that is generated when a ligand is allowed to act on it is also different among the aforementioned types of DC (Non-Patent Documents 20 and 21). Moreover, it has been reported that a large amount of IL-12p70 is generated with CpG+CD40L in PDC (Non-Patent Document 22). Thus, it is interesting to think about maturation of a DC subset in an actually living body, and more specifically, the type of a DC subset, the type of a natural immune response that stimulates such a DC subset, and the phenotype of DC that becomes mature from such a DC subset by the influence of a cytokine that acts in an autocrine/paracrine manner.

In 1990s, it became possible to differentiate and/or induce DC from precursor cells using a cytokine, and thus it became possible to treat a large amount of DC. As a result, it has been revealed that DC plays an important role in immune responses on the molecular, cellular, and biological levels. At the same time, DC has become a focus of attention as a target of immunosuppression. As important functions, DC receives a natural immune response, and at the same time, it incorporates an antigen into cells (phagocytosis) and then transfers information regarding the antigen to T cells, so as to stimulate and activate the T cells.

It may be no exaggeration to say that such a functional change in DC from ability to incorporate an antigen in the cells to ability to present the antigen to the T cells is responsible for actuation of the acquired immunity after receiving the aforementioned natural immune response. It is not difficult to image that a change in a membrane molecule presented on a cell surface occurs attended with the aforementioned functional change. However, if taking into consideration the phenomenon that occurs during such a maturation process, it can be said that expression of a molecule that is newly expressed on a cell membrane surface is not always caused by expression of mRNA. For example, it has been known that, HLA-DR, which is expressed even at an immature stage, is translocated from inside the cell onto the cell membrane surface, with almost no change in the expression levels of mRNA and a protein, as it becomes mature. Moreover, there may be cases where the actual expression level of mRNA has almost no correlation with the expression level of a protein when a comparison is made between them, or there may also be cases where mRNA is not translated as a protein, although mRNA is expressed. To date, DC has vigorously been analyzed, since it has been advantageous in that it can be amplified from the gene side, it can be analyzed in large quantities, it is easy to handle it, and various methods can be applied thereto. If it is possible to follow a change in an actually expressed protein, it can be said that such a change reflects well the intracellular phenomenon and thus it is close to the reality.

With regard to DC, in addition to the aforementioned findings, it has been revealed that DC has several subsets. It has been known that such subsets have different functions depending on whether they are mature or immature. However, the reason why such subsets have different functions is still unknown. It is desired to clarify a membrane molecule that may be involved in at least intracellular signaling, and in particular, to clarify a membrane molecule that is expressed together with maturation of DC.

It is anticipated that clarification of such a membrane molecule will bring on the following effects (1) to (4): (1) an antitumor effect using NKT cell activation due to activation of dendritic cells or CTL induction as a trigger; (2) the effect of preventing and/or controlling autoimmune disease or allergy based on a balance shift of Th1/Th2 attended with a functional change in dendritic cells; (3) the effect of preventing and/or suppressing virus or bacteria infectious disease by suppressing maturation of dendritic cells; and (4) the improvement of (1), (2) and (3) above, by eliminating activated PDC by *in vivo*/*ex vivo* depletion. Known examples of the phenomena and mechanisms described in (1) to (4) above include: activation of T cells by B7 family molecules; maturation of dendritic cells by CD40; and actuation of a natural immune system and an acquired immune system, using, as an entrance, a Toll-like receptor involved in an innate immune response. However, such phenomena do not entirely clarify the aforementioned membrane molecule.
[Non-Patent Document 1] Science, 284, 1313-1318 (1999)
[Non-Patent Document 2] Trends Cell Biol, 11, 304-311 (2001)
[Non-Patent Document 3] J Biol Chem, 274, 17406-17409 (1999)
[Non-Patent Document 4] J Immunol, 163, 2382-2386 (1999)
[Non-Patent Document 5] Science, 282, 2085-2088 (1998)
[Non-Patent Document 6] Nature, 410, 1099-1103 (2001)
[Non-Patent Document 7] Int Immunol, 13, 933-940 (2001)
[Non-Patent Document 8] Science, 303, 1529-1531 (2004)
[Non-Patent Document 9] Nature, 408, 740-745 (2000)
[Non-Patent Document 10] Immunity, 11, 115-122 (1999)
[Non-Patent Document 11] J Immunol, 166, 5688-5694 (2001)
[Non-Patent Document 12] Nat Immunol, 2, 835-841 (2001)
[Non-Patent Document 13] Nature, 413, 78-83 (2001)
[Non-Patent Document 14] J Immunol, 167, 5887-5894 (2001)
[Non-Patent Document 15] J Immunol, 164, 554-557 (2000)
[Non-Patent Document 16] Cell, 103, 909-918 (2000)
[Non-Patent Document 17] J Biol Chem, 276, 37692-37699 (2001)
[Non-Patent Document 18] J Immunol, 164, 4991-4996 (2000)
[Non-Patent Document 19] Nature, 410, 1103-1107 (2001)
[Non-Patent Document 20] J Exp Med, 194, 863-869 (2001)
[Non-Patent Document 21] Eur J Immunol, 31, 3388-3393 (2001)
[Non-Patent Document 22] Eur J Immunol, 31, 3026-3037 (2001)
[Non-Patent Document 23] J Leucocyte Biol, 59, 208-218 (1996)
[Non-Patent Document 24] Biochem Biophys Res Commun, 231, 1-6 (1997)
[Non-Patent Document 25] Science, 261, 609-612 (1993)
[Non-Patent Document 26] J Immunol, 161, 2762-2771 (1998)

### DISCLOSURE OF THE INVENTION

### Problems to be Solved by the Invention

It is an object of the present invention to identify a membrane molecule that is specifically expressed on activated plasmacytoid dendritic cells, for the purpose of preventing or improving affection or disease such as cancer, autoimmune disease, allergy, or infectious disease by regulating the functions of dendritic cells capable of integrating an immune system.

### Means for Solving the Problems

The present inventors have conducted intensive studies directed towards achieving the aforementioned object. The inventors have comprehensively identified cell surface molecules that are expressed on dendritic cells by a new cell membrane preparation method and cell membrane molecule identification method, named as "Plasma Membrane Focused Proteomics." As a result, the inventors have discovered a molecule that is specifically expressed on the cell membrane surface of activated mouse plasmacytoid dendritic cells (PDC). The aforementioned membrane molecule expressed in a mouse has been identified by bioinformatics, and the full-length cDNA thereof has been cloned. Thereafter, a monoclonal antibody that specifically recognizes the aforementioned membrane molecule has been established. Expression of the membrane molecule in various types of immunocompetent cells existing in mouse splenic cells was analyzed. As a result, it was found that expression of the membrane molecule was not confirmed in resting/naive leukocytes, but that when bulk leukocytes were stimulated with LPS, several CD11c positive cell populations expressed the present membrane molecule at a high level. The phenotype on the cell surface was examined more in detail. As a result, it was found that they were homogeneous cell populations of B220 positive, CD11b negative, Gr-1 positive, MHC class II positive, and B7-2 positive cells. From these results, it became clear that the aforementioned membrane molecule is specifically or dominantly expressed on activated plasmacytoid dendritic cells. Such activated plasmacytoid dendritic cells are known as cells that generate *in vivo* a large amount of type I interferon or IL-12, as a result of virus infection, and in particular, they are essential for antiviral action. In addition, it has been known that the concentration of type I interferon in blood is abnormally increased in patients with autoimmune diseases such as SLE or Crohn's disease. Thus, it has been reported that activation of PDC may trigger such autoimmune diseases. The findings of the present invention may clarify a novel immune mechanism and may become a key for new drug discovery.

The present invention provides a protein having any one of the following amino acid sequences:
(a) an amino acid sequence as shown in SEQ ID NO: 2;
(b) an amino acid sequence, which comprises a deletion, substitution, insertion and/or addition of one or several amino acid residues with respect to the amino acid sequence as shown in SEQ ID NO: 2, and which is capable of regulating the functions of dendritic cells; and
(c) an amino acid sequence, which has homology of 80% or more with the amino acid sequence as shown in SEQ ID NO: 2, and which is capable of regulating the functions of dendritic cells.

Another aspect of the present invention provides a regulator of the functions of dendritic cells, which comprises the aforementioned protein of the present invention. The regulator of the functions of dendritic cells of the present invention can be preferably used to increase generation of type I interferon.

Further another aspect of the present invention provides DNA encoding the aforementioned protein of the present invention.

Further another aspect of the present invention provides DNA having any one of the following nucleotide sequences:
(a) a nucleotide sequence as shown in SEQ ID NO: 1;
(b) a nucleotide sequence, which comprises a deletion, substitution, insertion and/or addition of one or several nucleotides with respect to the nucleotide sequence as shown in SEQ ID NO: 1, and which encodes an amino acid sequence capable of regulating the functions of dendritic cells; and
(c) a nucleotide sequence, which hybridizes with the nucleotide sequence as shown in SEQ ID NO: 1 or a complementary sequence thereof under stringent conditions, and which encodes an amino acid sequence capable of regulating the functions of dendritic cells.

Further another aspect of the present invention provides a recombinant vector, which comprises the aforementioned DNA of the present invention.

Further another aspect of the present invention provides a transformant, which comprises the aforementioned DNA or recombinant vector of the present invention.

Further another aspect of the present invention provides an antibody, which specifically immunologically binds to the aforementioned protein of the present invention or a fragment thereof.

Preferably, the antibody of the present invention immunologically recognizes plasmacytoid dendritic cells.

Preferably, the antibody of the present invention recognizes the extracellular region of the aforementioned protein of the present invention.

Preferably, the antibody of the present invention is either a polyclonal antibody or a monoclonal antibody.

Further another aspect of the present invention provides a method for separating human- or other animals-derived plasmacytoid dendritic cells by using the aforementioned antibody of the present invention.

Further another aspect of the present invention provides a method for detecting plasmacytoid dendritic cells by using the aforementioned antibody of the present invention.

### BEST MODE FOR CARRYING OUT THE INVENTION

The present invention will be described more in detail below.

### (1) Novel membrane molecule of the present invention and DNA encoding the same

As stated above, the present invention has been made based on the findings that expression of the membrane molecule (A) of the present invention is increased together with maturation of DC.

With regard to the mouse dendritic cell membrane molecule of the present invention, mouse bone marrow cells were cultured in the presence of GM-CSF to induce mouse bone marrow-derived immature dendritic cells (DC), and the thus induced mouse bone marrow-derived immature dendritic cells were then stimulated with lipopolysaccharide, so as to obtain mature DC based on the results of the stimulation. Thereafter, a cell membrane was prepared, and the soluble protein thereof was then subjected to methods such as concanavalin A sepharose chromatography, wheat germ agglutinin-sepharose chromatography, or SDS-polyacrylamide gel electrophoresis, so as to fractionate a membrane protein. The fractionated membrane protein was subjected to microanalysis using LC/MS (in particular, QTRAP manufactured by Applied Biosystems), so as to identify the mouse dendritic cell membrane molecule of the present invention (refer to Examples 1 to 5 as described later). Moreover, primers were synthesized based on a plurality of partial amino acid sequences identified by the LC/MS method. Thereafter, a polymerase chain reaction (PCR) was carried out using a cDNA library derived from mouse mature DC as a template, so as to amplify a gene fragment of the membrane molecule of the present invention. Using such a gene fragment as a probe, colony hybridization was carried out to select a clone comprising the gene of the membrane molecule (A) of the present invention. Thereafter, the nucleotide sequence thereof (SEQ ID NO: 1) and the amino acid sequence thereof (SEQ ID NO: 2) were determined (refer to Example 6 as described later).

As a result of hydropathy plot analysis [J. Exp. Med., Vol. 157, pp. 105-132, 1982] and signal sequence prediction analysis [Protein Eng., Vol. 10, pp. 1-6, 1997], it was suggested that the membrane molecule (A) of the present invention is a type I membrane protein having a signal sequence.

### Membrane molecule (A)

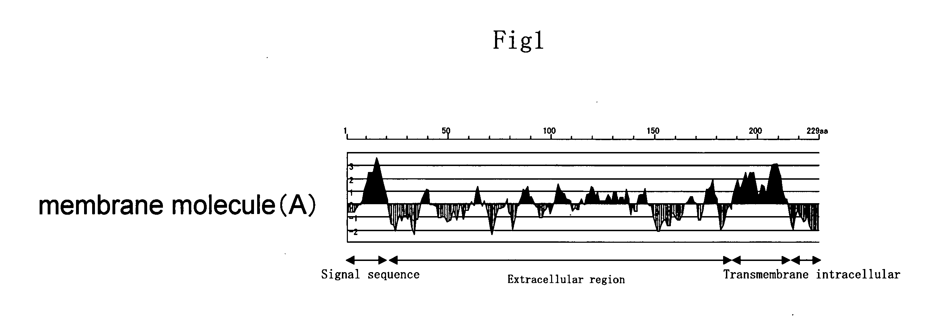

The present membrane molecule consists of 229 amino acid residues. From the results of hydropathy plot analysis (Figure 1) and signal sequence prediction analysis, it can be considered that the present membrane molecule consists of a signal sequence consisting of 23 amino acid residues, an extracellular region consisting of 169 amino acid residues, a transmembrane region consisting of 20 amino acid residues, and an intracellular region consisting of 17 amino acid residues. Moreover, from the results of homology search and motif search, it is predicted that the present membrane molecule has one Ig domain (IgV set) from the position of a cysteine (Cys) residue necessary for formation of a disulfide bond. Three potential asparagine-linked sugar chain-added portions are present (asparagine residues at positions 108, 109, and 179). Furthermore, since a lysine (Lys) residue is present in a trans-cell membrane region, it is predicted that the membrane molecule is associated with a certain adapter molecule via an ionic bond.

The membrane molecule group of the present invention can be synthesized in a large amount by utilizing gene cloning and DNA recombinant techniques. As common techniques used in the synthesis of a large amount of membrane molecule group, there can be used standard techniques described in, for example: Sambrook et al., Molecular Cloning: A Laboratory Manual, 2nd Ed., Cold Spring Harbor Laboratory Press, Cold Spring Harbor, N. Y (1989); Ausubel et al., Current Protocols in Molecular Biology, Greene Publishing Associates and Wiley Interscience, N. Y. (1989); etc.

From human mature DC, mRNA is extracted by the aforementioned standard techniques, so as to prepare a cDNA library. Using a specific probe synthesized based on the sequence as shown in SEQ ID NO: 1, the aforementioned library is screened, so as to obtain cDNA of interest. Otherwise, sense and antisense primers for amplifying a sequence comprising the mature sequence of a molecule of interest were synthesized based on the sequence as shown in SEQ ID NO: 1, and PCR is then carried out using the aforementioned cDNA library as a template, so as to amplify cDNA of interest. It is better that PCR be carried out using an automated thermal cycler. A single cycle of PCR reaction consists of DNA denaturation (e.g. 94°C, 15 to 30 seconds), primer annealing (e.g. 55°C, 30 seconds to 1 minute), and an elongation reaction (e.g. 72°C, 30 seconds to 10 minutes) in the coexistence of 4 types of substrates (dNTP), which are performed in the presence of heat stable polymerase (Taq, etc.), template DNA, and primers. The PCR reaction can be carried out for approximately 25 to 40 cycles, and the reaction product is then heated at 70°C to 75°C for 5 to 15 minutes. The size of a primer is generally at least 15 nucleotides.

By the aforementioned method, DNA encoding the membrane molecule (A) of the present invention can be cloned. A specific example of DNA encoding the membrane molecule (A) of the present invention is DNA having the nucleotide sequence as shown in SEQ ID NO: 1.

### Mutant

In addition to the aforementioned membrane molecule having the amino acid sequence as shown in SEQ ID NO: 2, the present invention also provides a protein having an amino acid sequence, which comprises a deletion, substitution, insertion and/or addition of one or several amino acid residues with respect to the amino acid sequence as shown in SEQ ID NO: 2 and which is capable of regulating the functions of dendritic cells, and a protein having an amino acid sequence, which has homology of 80% or more, preferably 90% or more, more preferably 95% or more, and most preferably 98% or more with the amino acid sequence as shown in SEQ ID NO: 2 and which is capable of regulating the functions of dendritic cells (hereinafter referred to also as a mutant protein).

The term "one or several" amino acid residues is used in the present specification to mean preferably 1 to 20, more preferably 1 to 10, further more preferably 1 to 8, particularly preferably 1 to 5, and most preferably 1 to 3 amino acid residues.

The term "homology" is used in the present specification to mean identity or similarity in sequence among two or more amino acid sequences or nucleotide sequences. Such sequences can be compared by common methods including a diagonal diagrammatic technique or a frequency distribution, for example.

The expression "capable of regulating the functions of dendritic cells" is used in the present specification to mean that the aforementioned mutant proteins have a function to regulate the functions of dendritic cells, which is equivalent to or substantially equivalent to the function of the natural membrane molecule (A) of the present invention. A specific example of such a function to regulate the functions of dendritic cells is a function to increase generation of type I interferon.

DNA encoding the aforementioned mutant proteins is also included in the scope of the present invention. Examples of such DNA include: DNA having a nucleotide sequence, which comprises a deletion, substitution, insertion and/or addition of one or several nucleotides with respect to the nucleotide sequence as shown in SEQ ID NO: 1 and which encodes an amino acid sequence capable of regulating an immune response; and DNA having a nucleotide sequence, which hybridizes with the nucleotide sequence as shown in SEQ ID NO: 1 or a complementary sequence thereof under stringent conditions and which encodes an amino acid sequence capable of regulating the functions of dendritic cells.

The term "one or several" nucleotides is used in the present specification to mean preferably 1 to 20, more preferably 1 to 10, further more preferably 1 to 8, particularly preferably 1 to 5, and most preferably 1 to 3 nucleotides.

The term "a nucleotide sequence, which hybridizes with ... under stringent conditions" is used in the present specification to mean a nucleotide sequence that is detected when hybridization is carried out under conditions consisting of 0.5 M NaHPO₄, 7% SDS, 1mM EDTA and 65°C, and washing is then carried out at 0.1 x SSC/0.1% SDS and 68°C, for example.

The mutant protein of the present invention may have all or a part of the functions of a natural membrane molecule. Any type of mutant may be within the scope of the present invention, as long as it has high homology (80% or more) with such a natural membrane molecule, and it has ability to regulate the functions of dendritic cells. Such a mutant can be produced by introducing a desired modification (deletion, substitution, insertion and/or addition) into a natural membrane molecule according to the site-directed mutagenesis, the PCR method, etc. (please refer to the aforementioned Molecular Cloning and Current Protocols in Molecular Biology). Examples of such a modification include substitution between conservative amino acids, such as substitution between acidic amino acids (aspartic acid and glutamic acid), between basic amino acids (lysine and arginine), or between hydrophobic amino acids (leucine, isoleucine, valine, etc.).

When the mutant of the present invention is obtained from a natural source or the like, a probe is produced based on the nucleotide sequence, and hybridization is then carried out under moderately or highly stringent conditions. Thereafter, a reaction product is washed under highly stringent conditions to obtain a gene encoding a mutant of interest. The thus obtained gene is incorporated into a suitable vector, and it is then allowed to express, thereby obtaining the mutant of interest. Examples of such highly stringent conditions include a hybridization in 0.5M NaHPO₄, 7% SDS, 1mM EDTA, and at 65°C, and the subsequent washing operation in 0.1 x SSC/0.1% SDS, and at 68°C (please refer to the aforementioned Current Protocols in Molecular Biology). Such hybridization conditions can be determined by selecting a temperature and an ionic strength, as appropriate. In general, as the temperature increases, or as the ionic strength decreases, stringency increases. Thus, persons skilled in the art are able to select appropriate hybridization conditions. The present invention also includes DNA encoding the aforementioned mutant, which has homology of 80% or more, preferably 90% or more, more preferably 95% or more, and most preferably 98% or more with the nucleotide sequence as shown in SEQ ID NO: 1.

Since the protein of the present invention (the membrane protein (A) of the present invention) is capable of regulating the functions of dendritic cells, it can be used as a regulator of the functions of dendritic cells. Thus, the protein of the present invention can be used to regulate the functions of dendritic cells and to increase generation of type I interferon, for example.

When the protein of the present invention is used as such a regulator of the functions of dendritic cells, a method of administering the protein of the present invention and a dosage form are not particularly limited. In general, it is administered via intravenous, intraarterial, intramuscular or oral administration, or via rectal administration in a suppository form. It can be orally or parenterally administered by the combined use of a pharmaceutically acceptable excipient or diluent. Parenteral administration is preferable. Such a regulator is applied once or divided over several administrations per day. The dosage is determined depending on conditions such as the severity, age, sex, and body weight of a patient. Such a dosage is preferably within a range that does not cause side effects, and it is generally between 0.1 µg/kg/day and 10 mg/kg/day.

### (2) Recombinant vector and transformant of the present invention

The DNA of the present invention can be inserted into a suitable vector, for its use. The type of a vector used in the present invention is not particularly limited. For example, the vector may be either an autonomously replicating vector (for example, a plasmid, etc.), or a vector that is incorporated into the genome of a host cell when it is introduced into the host cell, and is then replicated together with the incorporated chromosome.

The vector used in the present invention is preferably an expression vector. In such an expression vector, the gene of the present invention is functionally ligated to a suitable transcriptional/translational regulatory sequence (e.g. a promoter, etc.). Examples of an expression vector include a plasmid, a phage, a cosmid, and a virus. A promoter is a DNA sequence exhibiting a transcriptional activity in host cells. Such a promoter can be selected, as appropriate, depending on the type of a host.

The transcriptional/translational regulatory sequence may include a promoter and an enhancer that are selected depending on a host/vector system used. For example, when a bacterial system is used as a host, P_{L}, P_{R}, Ptrp, Plac, and the like can be used as promoters. When a yeast system is used as a host, PH05, GAP, ADH and AOX1 promoters, and the like can be used as promoters. When an animal cell is used as a host, an SV40 early promoter, a retrovirus promoter, a heat shock promoter, and the like can be used. Example of promoters which is operable in insect cell may include a polyhedrin promoter, a P10 promoter, an *Autographa californica* polyhedrosis basic protein promoter, a baculovirus immediate early gene 1 promoter, a baculovirus 39K delayed-early gene promoter, and the like.

In addition, the DNA of the present invention may functionally bind to a suitable terminator, as necessary. Moreover, the recombinant vector of the present invention may further comprise elements such as a polyadenylation signal (for example, those derived from SV40 or an adenovirus 5Elb region) or a transcription enhancer sequence (for example, an SV40 enhancer).

Furthermore, the recombinant vector of the present invention may further comprise a DNA sequence enabling replication of the above vector in host cells. An example of such a DNA sequence may be an SV40 replication origin (when host cells are mammalian cells).

Still further, the recombinant vector of the present invention may further comprise a selective marker. Examples of such a selective marker may include: genes whose complements are absent in host cells, such as a dihydrofolate reductase (DHFR) gene or a *Schizosaccharomyces pombe* TPI gene; and genes resistant to drugs such as ampicillin, kanamycin, tetracycline, chloramphenicol, neomycin, or hygromycin. A method for ligating the gene of the present invention, a promoter, and as desired, a terminator and/or a secretory signal sequence to one another, and inserting the ligated product into a suitable vector, is well known to persons skilled in the art.

Various expression vectors, the types of which depend on a host, have been commercially available or have been deposited. Otherwise, such expression vectors are described in publications such as documents, and they are available. For example, pQE (QIAGEN), pBluescript II SK+ (Stratagene), pET (Novagen), etc. can be used for bacteria.

A transformant can be produced by introducing the DNA or recombinant vector of the present invention into a suitable host.

Any types of cells may be used as host cells, into which the DNA or recombinant vector of the present invention is introduced, as long as they allow the protein of the present invention to be express therein. Examples of such host cells used herein may include prokaryotic cells, yeast cells, animal cells, fungal cells, insect cells, and plant cells.

Examples of prokaryotic cells may include cells belonging to genus Escherichia, genus Bacillus, and genus Pseudomonas. Such cells may be transformed by the protoplast method, or other known methods using competent cells.

Examples of yeast cells may include cells belonging to genus Saccharomyces, genus Schizosaccharomyces, and genus Pichia. Specific examples may include *Saccharomyces cerevisiae* and *Saccharomyces kluyveri.* Examples of a method of introducing a recombinant vector into a yeast host may include electroporation, the spheroplast method, and the lithium acetate method.

Examples of animal cells may include human embryonic kidney cells, human leukemia cells, African green monkey kidney cells, and Chinese hamster ovary cells (CHO). A method of transforming mammalian cells to allow a DNA sequence introduced into the cells to express has also been known. Examples of such a method used herein may include electroporation, the calcium phosphate method, and the lipofection method.

Examples of fugal cells may include cells belonging to filamentous fungi, such as Aspergillus, Neurospora, Fusarium, or Trichoderma. When filamentous fungi are used as host cells, transformation can be carried out by incorporating a DNA construction into the chromosome of a host, so as to obtain recombinant host cells. Such incorporation of a DNA construction into a host chromosome can be carried out in accordance with known methods, such as homologous recombination or heterologous recombination.

When insect cells are used as host cells, a recombinant gene-introduced vector and a baculovirus are co-introduced into the insect cells, so as to obtain a recombinant virus in a supernatant of cultured insect cells. Thereafter, insect cells are infected with such a recombinant virus, so as to allow a protein to express therein (which is described, for example, in Baculovirus Expression Vectors, A Laboratory Manual; and Current Protocols in Molecular Biology, Bio/Technology, 6, 47 (1988)).

For example, an *Autographa californica* nuclear polyhedrosis virus, with which insects belonging to *Mamestra brassicae* are infected, can be used as a baculovirus.

Examples of insect cells may include: Sf9 and Sf21, ovarian cells of *Spodoptera frugiperda* [Baculovirus Expression Vectors, A Laboratory Manual, W. H. Freeman and Company, New York, (1992)]; and HiFive (manufactured by Invitrogen), ovarian cells of Trichoplusia ni.

Examples of a method of co-introducing a recombinant gene-introduced vector and the aforementioned baculovirus into insect cells, so as to prepare a recombinant virus, may include the calcium phosphate method and the lipofection method.

The transformed or transfected host cells are cultured in a suitable culture medium, so as to allow the gene of interest to express. Thereafter, the generated membrane molecule of the present invention is recovered from the medium or the host cells. When the present membrane molecule is recovered from the cells, after completion of the culture, the cells are separated by centrifugation or the like, and they are then suspended in an aqueous buffer. Thereafter, the cells are crushed by a sonic treatment, French press, Dyno mill, etc., so as to obtain a cell-free extract. The membrane molecule can be isolated and purified by combining common methods applied in purification of proteins, such as gel filtration, various types of chromatography including ion exchange chromatography, affinity chromatography, hydrophobic chromatography and the like, HPLC, electrophoresis, salting-out, and the organic solvent precipitation method.

### (3) Antibody of the present invention

The antibody of the present invention is an antibody which specifically immunologically binds to the protein of the present invention described in (1) above or a fragment thereof.

The expression "specifically immunologically bind to" relating to the antibody of the present invention is used in the present specification to mean that the antibody of the present invention immunologically cross-reacts with an epitope, which only the present membrane molecule has, and that it does not cross-react with other proteins such as a TREM family. Such an epitope can be selected by aligning the amino acid sequence of the membrane molecule of the present invention with the amino acid sequence of another protein for comparison, so as to select an essentially different sequence portion (contiguous, at least 5 amino acids, preferably at least 8 amino acids, and more preferably at least 15 amino acids), for example.

All types of antibodies having the aforementioned characteristics are included in the present invention. An antigenic epitope used to obtain an antibody of interest can be selected from a region having high antigenecity, a superficial region, a region that may be not have a secondary structure, or a region showing no homology or low homology with other proteins (in particular, other proteins in a family, to which each membrane molecule belongs), in the amino acid sequences of the present membrane molecule group. Herein, such a region having high antigenecity can be estimated by the method of Parker et al. [Biochemistry, Vol. 25, pp. 5425-5432 (1986)]. Such a superficial region can be estimated by calculating a hydropathy indexes and plotting them, for example. Such a region that may be not have a secondary structure can be estimated by the method of Chou and Fasman [Adv. Enzymol. Relat. Areas Mol. Biol., Vol. 47, pp. 45-148 (1978)], for example. Moreover, such a region showing no homology or low homology with, in particular, other proteins in a family, to which each membrane molecule belongs, can be estimated by homologous comparison between the amino acid sequence of each membrane molecule and the amino acid sequence of another protein.

Based on the partial amino acid sequence of the present membrane molecule estimated by the aforementioned methods, a peptide having the aforementioned amino acid sequence can be synthesized by a peptide synthesis method. The peptide of interest is synthesized, using a commercially available peptide synthesizer based on solid-phase peptide synthesis, which was developed by the method of R. B. Merrifield [Science, Vol. 232, pp. 341-347 (1986)], for example. After dissociation of a protecting group, the synthesize peptide is purified by a single use or the combined use of methods such as ion exchange chromatography, gel filtration chromatography, or reverse phase chromatography. The purified peptide is allowed to bind to a carrier protein such as keyhole limpet hemocyanin (KLH) or albumin, so that it can be used as an immunogen.

Furthermore, a polyclonal antibody or a monoclonal antibody reacting with the present membrane molecule can be produced by a known method using, as an immunogen, the gene recombinant membrane molecule of the invention. In this case, the term "recombinant" used for the present membrane molecule, a monoclonal antibody, a polyclonal antibody, or other proteins, means that such proteins are produced using recombinant DNA in host cells. As such host cells, either prokaryotes (e.g. bacteria such as *Escherichia coli*) or eukaryotes (e.g. yeasts, CHO cells, insect cells, etc.) can be used.

The "antibody" of the present invention may be any one of a peptide antibody, a polyclonal antibody, and a monoclonal antibody. Such an "antibody" can be obtained by immunizing a mouse or other suitable host animals with an antigen or an antigen-expressing cell via a subcutaneous, intraperitoneal, or intramuscular route, so as to obtain lymphocytes that generate or may generate an antibody that specifically binds to the protein used in the immunization. Further, an antigen or an antigen-expressing cell may be administered to a transgenic animal having a repertoire of human antibody genes, used as a host animal, so as to obtain a desired human antibody [please refer to Proc. Natl. Acad. Sci. USA, Vol. 97, pp. 722-727 (2000), and International Publications WO96/33735, WO97/07671, WO97/13852 and WO98/37757]. Otherwise, lymphocytes may be immunized *in vitro.* A fraction binding to an antigen is collected from a serum obtained from a host animal, and it is then purified, so as to obtain a polyclonal antibody. Moreover, using a suitable fusion reagent such as polyethylene glycol, lymphocytes are fused with myeloma cells to form hybridoma cells, so as to produce a monoclonal antibody (Goding, Monoclonal Antibodies: Principals and Practice, pp. 59-103, Academic press, 1986). For example, the monoclonal antibody of the present invention can also be produced by applying a hybridoma method [Nature, Vol. 256, p. 495 (1975)] or a recombinant DNA method (Cabilly et al., US Patent No. 4,816,567).

An antigenic protein can be prepared by allowing DNA encoding the entire or partial sequence of the protein of the present membrane molecule to express in *Escherichia coli,* yeast, insect cells, animal cells, etc. The gene recombinant membrane molecule of the present invention is purified by a single use or the combined use of methods such as affinity chromatography, ion exchange chromatography, gel filtration chromatography, or reverse phase chromatography. The thus purified sample is used as an immunogen.

In addition, the antibody of the present invention may be either an intact antibody, or an antibody fragment such as (Fab')₂ or Fab.

Moreover, the antibody of the present invention also includes: a chimeric antibody obtained by substituting the constant region with a human constant region (e.g. a mouse-human chimeric antibody; Cabilly et al., US Patent No. 4816567, and Morrison et al., Proc. Natl. Acad. Sci. USA, Vol. 89, p. 6851 (1984)); and a humanized antibody obtained by substituting all the variable regions other than the constant region and the hypervariable region (or Complementary-determining region; CDR) with human sequences (Carter et al., Proc. Natl. Acad. Sci. USA, Vol. 89, p. 4285, 1992; and Carter et al., BioTechnology, Vol. 10, p. 163 1992).

Furthermore, an antibody reacting with the thus obtained antibody of the present invention, namely, an anti-ideotype antibody is also included in the scope of the present invention.

The thus obtained various types of antibodies reacting with the present membrane molecule can be used for various purposes, so as to make full use of the characteristics thereof. Utilizing the fact that the present membrane molecule is specifically expressed on mature DC and activated plasmacytoid dendritic cells, the present antibody is labeled with a fluorescent substance (rhodamine, fluorescamine, etc.), and the DC and plasmacytoid dendritic cells of interest can be detected and/or separated by publicly known FACS, or differentiation of mo-DC *in vitro* can be confirmed. Further, since the present membrane molecule is a protein whose expression level is increased in mature DC and activated plasmacytoid dendritic cells, it is also possible to separate immature DC from mature DC or to separate activated plasmacytoid dendritic cells by FACS using the present antibody. Detection of the present membrane molecule is not limited to detection by FACS. For example, it is predicted that the present membrane molecule will be detected by allowing the present antibody used as a primary antibody to act thereon in Western blotting, or expression of the present membrane molecule can be confirmed at a protein level. Further, the present antibody is allowed to bind to a solid phase (polystyrene beads, microtiter well surface, latex beads, etc.), and an immunological reaction is carried out in a heterogeneous system or a homogeneous system, so that a homologous membrane molecule can be detected and assayed (using methods such as a fluorescent antibody technique, ELISA, or radioimmunoassay). In this case, such an immunological reaction may be either a competitive reaction or a noncompetitive reaction. Moreover, a sandwich method using two or more antibodies (monoclones or polyclones) can also be applied to such an immunological reaction. For the aforementioned detection and assay, any types of immunological methods known in the present field can be applied.

Furthermore, the present antibody can also be used for the purpose of evaluating the functions of the present membrane molecule. Mature DC is strong APC. It has been known that mature DC stimulates and activates CD4 positive T cells via MHC class II molecules, and that it also stimulates and activates CD8 positive cytotoxic T cells via MHC class I molecules. In order to confirm whether or not such functions can be controlled, the present antibody can also be used in *in vitro* assays for confirming whether or not the functions in an allogenic MLR (mixed lymphocyte reaction) can be suppressed, whether or not the functions can be suppressed when CTL is antigen-specifically induced, whether or not it is a molecule involved in antigen presentation of DC, etc.

The antibody of the present invention can further be used to control an immune response *in vivo.* As stated above, it is predicted that the present membrane molecule group will be involved in co-stimulation and will also be involved in activation of T cells. Thus, if the antibody of the present invention is an antibody that inhibits the binding of the present membrane molecule group to a ligand on a T cell, it is anticipated that the present antibody will suppress activation of such T cells. Thus, it is anticipated that the functions of DC and also the functions of T cells can be controlled by allowing such an antibody capable of controlling the functions of the present membrane molecule group to act, thereby controlling immune responses.

Furthermore, it is also anticipated that the solubilized molecules of the present membrane molecule and the ligand of the present membrane molecule (that is, molecules corresponding to an extracellular region), and an antibody reacting with such molecules will also have the activity of controlling immune responses.

It is also possible to obtain the ligand of the present membrane molecule using the antibodies of the present invention or the solubilized molecules of the present membrane molecules. The ligand of the solubilized membrane molecule of the present invention directly acts on the present membrane molecule, so as to control a signal via the present membrane molecule on DC. In addition, a low molecular weight substance capable of modulating the interaction between the ligand of the present membrane molecule and the present membrane molecule and a low molecular weight substance capable of modulating an intracellular signal pathway associated with the ligand of the present membrane molecule and the present membrane molecule, are also useful for the control of signals.

When the antibodies of the present invention, the solubilized molecules of the present membrane molecules, the solubilized molecules of the ligands of the present membrane molecules, or the aforementioned low molecular weight substances are used in treatments, they can be applied to the treatments of diseases such as cancer, autoimmune disease, organ transplantation, infectious disease, or allergy.

The administration method and the dosage form are not particularly limited. The present membrane molecule, antibody, or the like can be administered via intravenous, intraarterial, intramuscular or oral administration, or via rectal administration in a suppository form. It is combined with a pharmaceutically acceptable excipient or diluent, and the mixed agent can be administered orally or parenterally. Parenteral administration is preferable. The agent is applied once or divided over several administrations per day. The applied dose is determined depending on conditions such as the severity, age, sex, and body weight of a patient, and the like. It is determined within a range that does not cause side effects.

The present invention will be described in the following examples. However, these examples are not intended to limit the scope of the present invention.

### EXAMPLES

The present invention will be described in the following examples. However, these examples are not intended to limit the scope of the present invention.

### Example 1 Preparation of cell membrane of DC

A cell membrane protein is problematic in terms of its originally low expression level and difficulty in handling it. As a means for solving such problems, it is necessary to establish a method for obtaining a cell membrane with a high purity. A cell membrane with a high purity was prepared by a method which comprises coating the surface of a cell membrane and disrupting uniformly the coated surface, so as to obtain a cell membrane with a higher specific gravity by density gradient centrifugation [J. Biol. Chem., Vol. 258, pp. 10062-10072 (1983)].

Taking into consideration a low expression level of target membrane molecule and difficulty in handling it, an attempt was made to collect a large amount of mouse DC. That is, bone marrow cells were cultured in the presence of GM-CSF to prepare a large amount of bone marrow cell-derived immature DC. A certain number of cells were stimulated with LPS (lipopolysaccharide), and the same number of cells were not stimulated with LPS. Thus, immature DCs and mature DCs were obtained in the same numbers (5 x 10⁸ cells).

### Example 2 High sensitivity detection of protein and in-gel digestion

A protein was detected by silver staining according to the technique of Mann et al. [Anal. Chem., Vol. 68, pp. 850-858, 1996]. The in-gel digestion method [Anal. Chem., Vol. 224, pp. 451-455, 1995] using trypsin was performed as an enzymatic digestion method to obtain analytical samples to be used below.

### Example 3 Fractionation of membrane protein

A cell membrane protein was solubilized from the cell membrane obtained in Example 1 using a surfactant. Thereafter, the cell membrane protein was applied to concanavalin A sepharose, and it was then washed with a surfactant-containing buffer. Such a pass-through fraction was named as ConA FT. The adsorbed fraction was eluted with a buffer that contained methyl-alpha-D-glucopyranoside and a surfactant (ConA EL). ConA FT was applied again to wheat germ agglutinin sepharose, and it was then washed with a surfactant-containing buffer. Such a pass-through fraction was named as WGA FT. The adsorbed fraction was eluted with a buffer that contained N-acetylglucosamine and a surfactant (WGA EL). The thus obtained ConA EL, WGA EL and WGA FT were used as samples in molecular identification. After these fractions had been applied to SDS-PAGE, a protein was detected by the method described in Example 2. A gel that was cut into a strip shape was subjected to in-gel digestion, so as to prepare an analytical sample.

### Example 4 Microanalysis by LC/MS

The sample obtained in Example 3 was analyzed using LC/MS (QTRAP, manufactured by Applied Biosystems). The sample was subjected to a PepMap reversed phase column (0.075 mm in internal diameter x 150 mm in length) (manufactured by LC packings) that had been equilibrated with 95% solution A (0.1% formic acid) and 5% solution B (0.08% formic acid, 80% acetonitrile). It was washed at a flow rate of 180 nl/minute for 5 minutes. Thereafter, the resultant was eluted successively by linearly increasing the proportion of solution B up to 50% for 67.5 minutes, and it was then introduced into MS. Data regarding the sample introduced into MS was acquired in the following repeated cycles, and thus the sequence information thereof was obtained. 1. Full MS Scan: Observation of molecular weight of parent ion with a range of m/z = 0 to 2000; 2. Zoom MS Scan: Observation of the valence of parent ion identified with Full MS Scan; 3. MS/MS Scan: Observation of daughter ion generated when He gas was applied to the molecule measured by Zoom MS Scan. Identification was performed by an identification method (SEQUEST algorithm), which scores and ranks the number of sequences that match with theoretical b,y series and the matching strength [American Society for Mass Spectrometry, Vol. 5, pp. 976-989, 1994].

### Example 5 Identification of the present membrane molecule

Among the molecules identified by the method described in Example 4, the present membrane molecule was identified as a novel molecule. Fragments subjected to identification were a divalent ion at m/z = 649.33 and a divalent ion at m/z = 749.91. The MS/MS patterns were attributed extremely well to two partial amino acid sequences of the present membrane molecule (11 residues, IDNLCYPFVSK (SEQ ID NO: 3), and 15 residues, GSSVVSTPDIIPATR (SEQ ID NO: 4)). As a result of the homology search of these partial sequences with respect to the non-redundant DNA database, it became clear that no other nucleotide sequences having these partial sequences were present.

### Example 6 Cloning of gene of the present membrane molecule

The partial amino acid sequences (SEQ ID NOS: 3 and 4) identified in Example 5 were searched through the mouse genome database. As a result, it was found that they were genes having partial sequences, wherein 3 different types of cDNA portions, AK080114, AK089248 and AK089332, which seemed to encode a single gene, were completely identical to one another. Thus, primers were synthesized based on gene sequences corresponding to the partial amino acid sequences discovered in Example 5, and RT-PCR was then carried out using a mouse mature DC cDNA library as a template. Primers used herein were of sizes of 22 mer and 25 mer, respectively. That is, there were used a sense primer, 5'-CTCCCCTCCTTTCTTGCTCAAC-3' (SEQ ID NO: 5), and an antisense primer, 5'-TCAGGAGTTACTGTTGTGTGCCTTC-3' (SEQ ID NO: 6). As a result, a gene fragment of the present membrane molecule of approximatley 800 bp or shorter was obtained. Colony hybridization was performed using the gene fragment as a probe, and a clone containing the gene of the present membrane molecule was selected, thereby determining the nucleotide sequence. The deduced open reading frame structure is as shown in SEQ ID NO: 2, and the deduced amino acid sequence is as shown in SEQ ID NO: 1. There have been no known genes completely identical to the obtained gene sequence.

The primary structure of the deduced amino acid sequence of the present membrane molecule was subjected to a hydropathy plot analysis according to the method of Kyte and Doolittle (J. Exp. Med., Vol. 157, pp. 105-132, 1982) (Fig. 1). As a result, it became clear that the present membrane molecule was a type I trans-cell membrane protein having a signal sequence at the N-terminus. The present membrane molecule (A) consisted of 229 amino acid residues. From the results of the hydropathy plot analysis, it was predicted that the present membrane molecule would comprise a signal sequence consisting of 23 amino acid residues, an extracellular region consisting of 169 amino acid residues, a transmembrane region consisting of 20 amino acid residues, and an intracellular region consisting of 17 amino acid residues. Moreover, from the results of homology search and motif search, it became clear that the extracellular region has three asparagine-linked sugar chain-addition sites and one immunoglobulin-like structure having cysteine residues necessary for formation of two immunoglobulin (Ig) domains. Furthermore, it was assumed that this extracellular Ig domain would have a Vset structure. Still further, the membrane molecule (A) did not have a motif capable of transferring a signal to the intracellular region, but it had a lysine residue in the trasmembrane region. Thus, it was predicted that the membrane molecule (A) would be associated with an adaptor molecule, and that the aforementioned membrane molecule would transfer a signal via such an adaptor molecule.

### Example 7 Production of solubilized recombinant form of the present membrane molecule

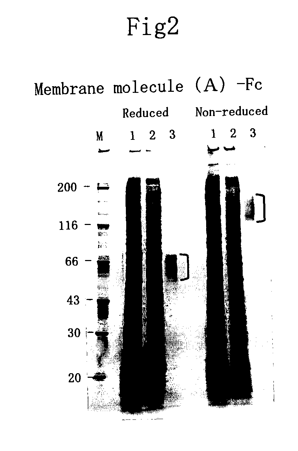

From the results of the hydropathy plot analysis (Figure 2), a region that was considered to be an extracellular domain was allowed to bind to the Fc region of human IgG1, so as to produce a recombinant form. In order to prevent the Fc region of human IgG1 from binding to Fc gamma receptors existing on the surfaces of various types of cells, amino acid substitution was conducted at 3 sites of the Fc region. Specifically, as an extracellular region, the amino acids to postion 192 of SEQ ID NO: 1 of (A) were selected. The amino acid sequence of the solubilized recombinant form is as shown in SEQ ID NO: 7. The sites subjected to amino acid substitution in the Fc region corresponded to position 212 (Ala→Leu), position 213 (Glu→Leu) and position 379 (Asp→Val) of the amino acid sequence as shown in SEQ ID NO: 7. This gene was allowed to transiently express in 293T cells under the control of a CMV promoter. Thereafter, an expression product was purified from the obtained culture supernatant by affinity chromatography using a resin, to which protien A having bindability to the Fc region had been bound (Figure 2).

### Example 8 Establishment of monoclonal antibody specific for the present membrane molecule

A rat was immunized with the recombinant form obtained in Example 7. Thereafter, cells obtained from the immunized rat were fused with cancer cells, so as to establish specific monoclonal antibody-generating hybridomas. For screening, the recombinant form described in Example 7 was allowed to bind to a solid phase, and clones having reactivity were selected by the ELISA method. At the same time, reactivity with cells, in which the aforementioned membrane molecule (A) had been forced to express, was analyzed with a flow cytometer (manufactured by BD Bioscience), thereby establishing a specific monoclonal antibody.

Specifically, the recombinant form described in Example 7 was mixed with a complete Freund's adjuvant to produce an emulsion, and 1 mg of the emulsion was then administered into the abdominal cavity of a Whister rat. Fourteen days after the immunization, the spleen was excised, and splenic cells were separated. The obtained splenic cells and myeloma cells were suspended in polyethylene glycol, so as to fuse the two types of cells. Thereafter, the mixed cells were cultured in an HAT medium for selection. The generated hybridomas were subjected to ultradilution to obtain monoclones. The aforementioned screening was performed on the supernatant of each monoclone, thereby establishing hybridomas generating monoclonal antibodies having reactivity with the aforementioned membrane molecule (A). Thereafter, a monoclonal antibody was purified from a serum free culture supernatant of the hybridomas by affinity purification against protein G.

### Example 9 Identification of adaptor molecule associateid with the present membrane molecule

The membrane molecule (A) had a lysine residue in the trans-cell membrane region. Thus, it was suggested that the membrane molecule (A) be associated with an adaptor molecule via an ionic bond. Hence, using the monoclonal antibody established by the method described in Example 8, such an adaptor molecule associated with the membrane molecule (A) was identified with a flow cytometer.

Specifically, the membrane molecule (A), downstream of which IRES-GFP was ligated, and the full-length gene of each adaptor molecule were inserted into an expression vector, and 293T cells were then co-transfected with the expression vector by the lipofection method using FuGENE6 (manufactured by Roche). Six to Twenty-four hours after the transfection, the 293T cells were analyzed using a flow cytometer (manufactured by BD Bioscience), in terms of expression of GFP-positive membrane molecule (A) on the cell surface.

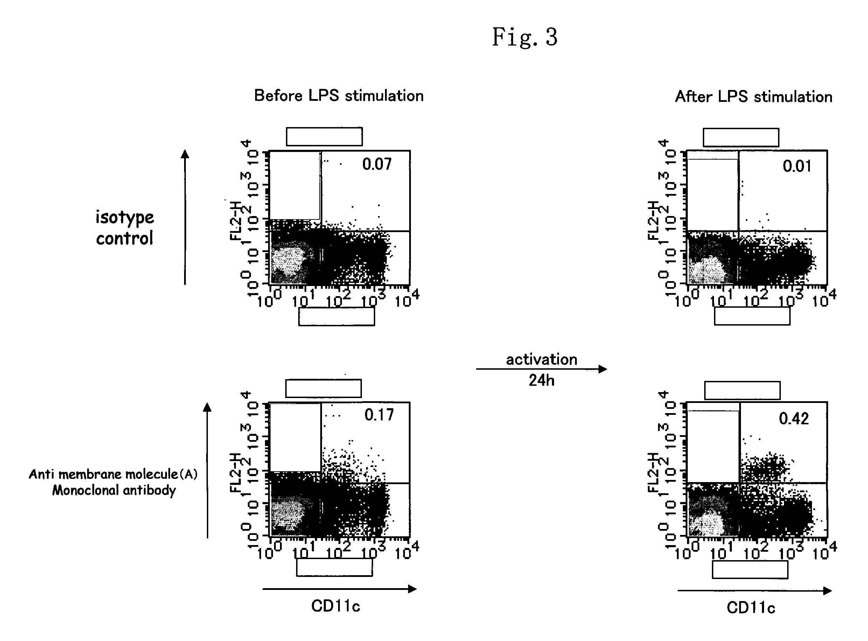

As a result, it was confirmed that a single membrane molecule (A) was not expressed on the cell surface, but that it was expressed on the cell membrane surface when it was allowed to co-express with an adaptor molecule DAP12 or DAP10 (Figure 3). This result strongly suggested that the adaptor molecule DAP12 or DAP 10 would be essential for expression of the membrane molecule (A) on the cell surface and signaling from the membrane molecule (A).

### Example 10 Analysis of reactivity of monoclonal antibody with mouse splenic cells

Localization of expression of the membrane molecule (A) was confirmed using the monoclonal antibody established by the technique described in Example 8. The aforementioned monoclonal antibody was biotinylated, and the biotinylated antibody was then allowed to act on mouse spleen-derived leukocytes. Phycoerythrin-bound avidin was allowed to react therewith to confirm membrane molecule (A)-expressing cells.

Specifically, the spleen was excised from a B6 mouse, and leukocytes were then prepared. The cells were stimulated with *Escherichia coli*-derived roughly purified lipopolysaccharide (manufactured by Sigma) for 24 hours. For biotinylation of a monoclonal antibody, such a monoclonal antibody was reacted with Sulfo-NHS-AC-biotin (manufactured by Dojindo Labolatories) in 20 mM Hepes-NaOH (pH 8.5) in ice for 1 hour for biotin labeling. Expression of the membrane molecule (A) in unstimulated splenic cells and in stimulated splenic cells was analyzed with a flow cytometer (manufactured by BD Bioscience), using a biotin-labeled monoclonal antibodies.

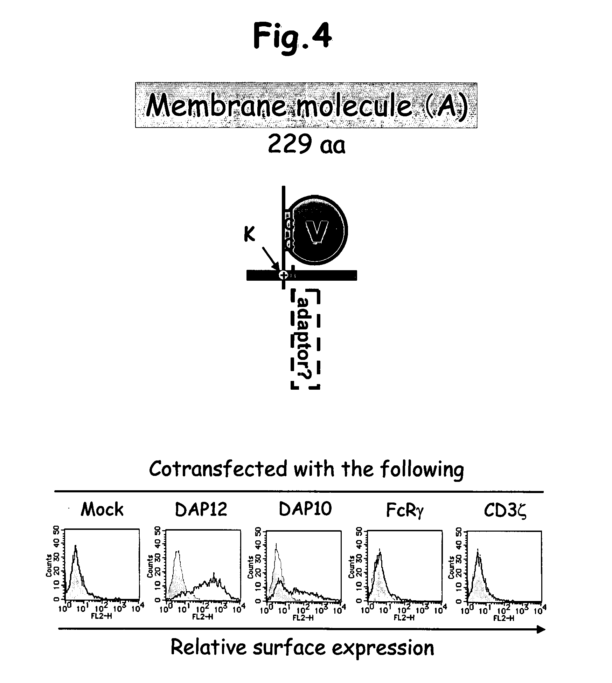

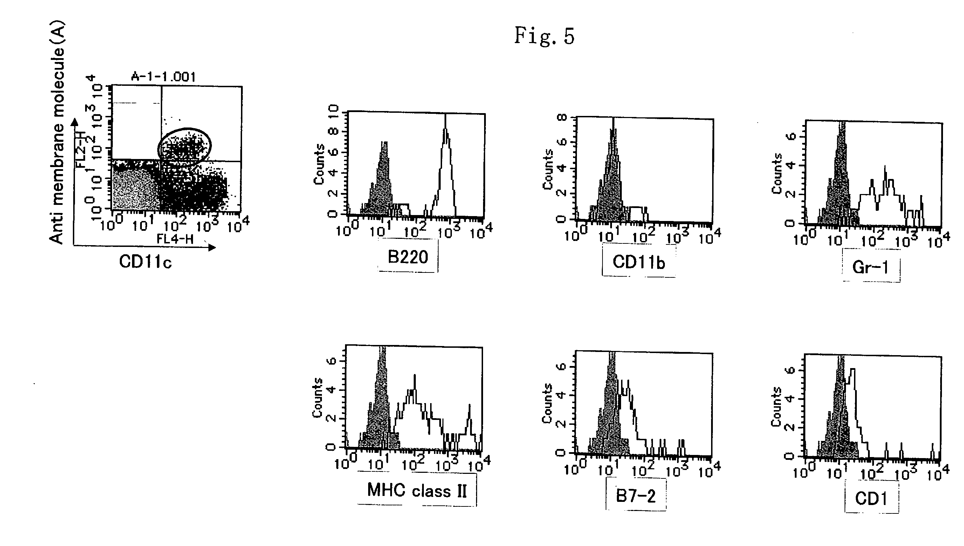

Cells having reactivity could not found in the unstimulated cells. On the other hand, in cells obtained by activating mouse spleen-derived leukocytes for 24 hours, it was confirmed that several CD11c positive cells had reactivity (Figure 4). In order to discover a cell population having reactivity with the monoclonal antibody, a further detailed analysis of the cell surface marker was conducted. As a result, it became clear that the cell population was a population of cells that were positive to B220, negative to CD11b, and positive to Gr-1, and it was also revealed that the present cells are plasmacytoid dendritic cells (PDC) (Figure 5). Moreover, the present cells were positive to MHC class II, B7-2, and CD1, and thus it was also revealed that the cells were mature. Accordingly, it was clarified that the membrane molecule (A) is a cell surface molecule that is expressed specifically for mature PDC. Such a molecule has been unknown so far.

### Example 11 Search for factor for increasing expression of membrane molecule (A)

As described in Example 9, it became clear that the present membrane molecule is expressed specifically for activated plasmacytoid dendritic cells. Thus, a factor for activating such plasmacytoid dendritic cells causing an increase in the expression of the membrane molecule (A) was searched. CD11c-positive, PDCA-1-positive, and B220-positive plasmacytoid dendritic cells were prepared from splenic cells by cencetration with MACS beads and sorting with a flow cytometer.

Specifically, splenic cells were allowed to react with PDCA-1 beads (manufactured by Miltenyi Biotec), and PDCA-1-positive cells were concentrated using an LS column (manufactured by Miltenyi Biotec). Thereafter, the cells were stained with APC-labeled CD11c, PE-labeled PDCA-1, and FITC-labeled B220. Subsequently, CD11c-positive, PDCA-1-positive, and B220-positive plasmacytoid dendritic cells were prepared by sorting using FACS Vantage SE (manufactured by BD Bioscience).

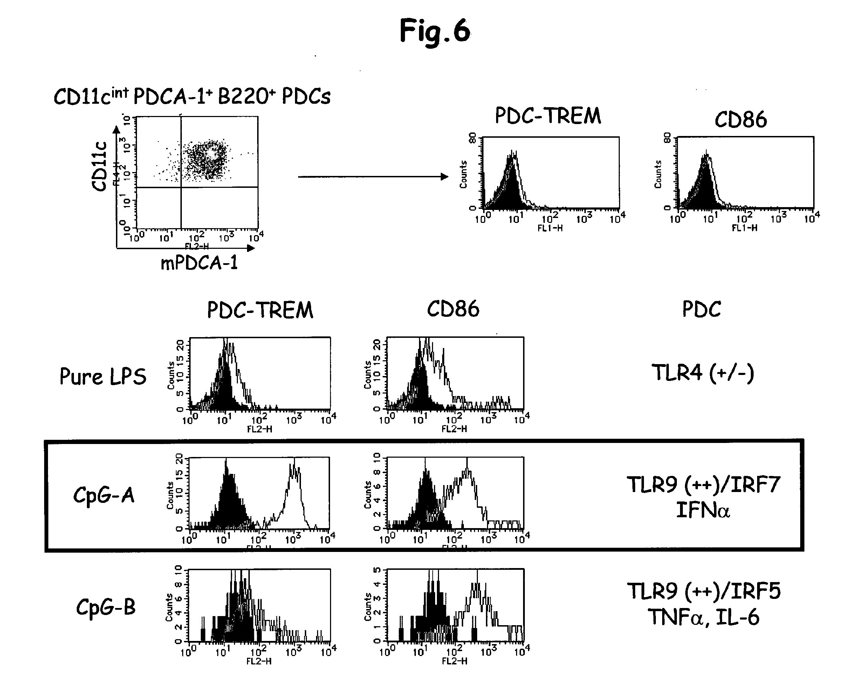

As a result of searching through various types of natural immunoactive substances, it was found that, among CpG members that cause activation via the signaling pathway of Toll-like Receptor 9 (TLR9), a group called CpG-A causes an increase in the expression of the membrane molecule (A) (Figure 6). As such CpG-A, a sequence called D19 (SEQ ID NO: 8) (ggTGCATCGATGCAgggggG; lower-case letters indicate a phosphothioate bond, CG at positions 8 and 9 indicate a phosphodiester bond, and CG at positions 8 and 9 indicate a CpG motif, and the underline indicates a palindrome) was used. Among various types of CpG-ODN members, CpG-A is a group that acts on PDC so as to allow it to generate a large amount of type I interferon (IFN). Thus, it was strongly suggested that the membrane molecule (A) would be associated with the function to generate a large amount of type I IFN from PDC.

### Example 12 Change in function of PDC by action of agonistic antibody against membrane molecule (A)

It has been known that PDC could be induced by culturing bone marrow cells in the presence of an Flt3 ligand (bone marrow cell-derived PDC). Such bone marrow cell-derived PDC which was induced using such a system was stimulated with CpG-A to obtain activated PDC. As described in Example 9, it became clear that the membrane molecule (A) transmits a downstream signal, using DAP 12 as an adaptor molecule. It has been known that one type of MAP kinase, pERK1/2, is present on a cascade at the downstream side. A monoclonal antibody reacting with the obtained membrane molecule (A) was allowed to act on the activated PDC, and agonistic activity was searched using phosphorylation of pERK1/2 as an indicator.

Specifically, bone marrow cells were collected from the femur of a mouse, and they were then cultured in the presence of a Flt3 ligand (25 ng/ml) for 10 days to obtain bone marrow cell-derived DC. This DC fraction was stained with APC-labeled CD11c, PE-labeled PDCA-1, and FITC-labeled B220. Thereafter, CD11c-positive, PDCA-1-positive, and B220-positive, bone marrow cell-derived plasmacytoid dendritic cells were sorted using FACS Vantage SE (manufactured by BD Bioscience). The obtained bone marrow cell-derived plasmacytoid dendritic cells were stimulated with CpG-A (1 ug/ml), and cells were prepared in course of time. The cells were applied to SDS-PAGE, and were then transcribed on a PVDF membrane. Thereafter, expression of pERK1/2 and phosphorylation thereof were confirmed by the Western blotting method using a specific antibody (manufactred by Cell Signaling).

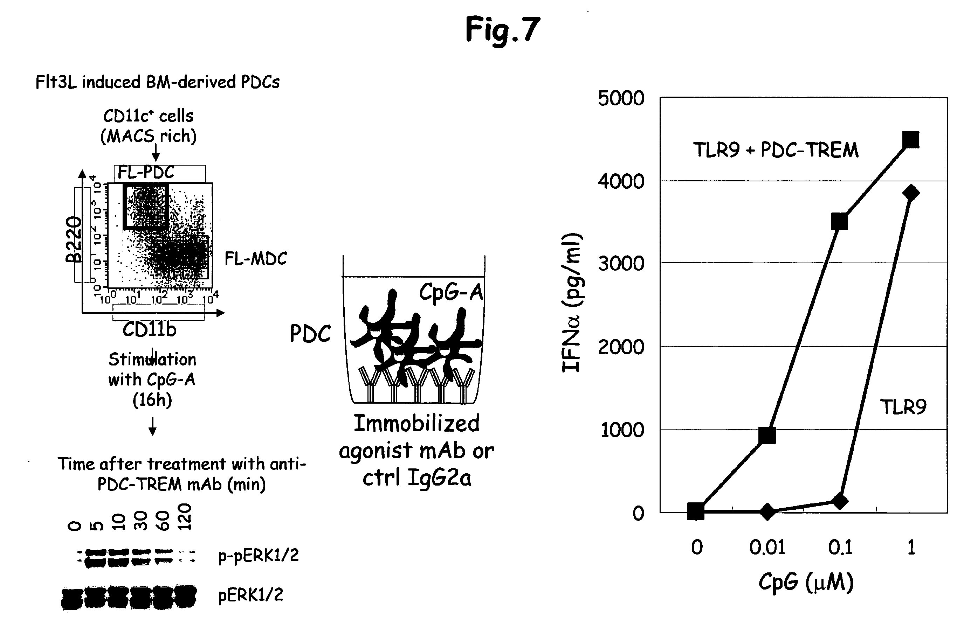

As a result, it was found that a monoclonal antibody having agonistic activity existed (Figure 7). Subsequently, spleen-derived PDC was cultured in the presence of various concentrations of CpG-A on a plate, on which such an agonistic monoclonal antibody reacting with the membrane molecule (A) had been immobilized. As a result, it was found that generation of type I IFN was promoted by a signal from the membrane molecule (A) (Figure 7). In particular, in the case of stimulation with a low concentration of CpG, the amount of type I IFN generated was 10 to 100 times increased. Considering virus infection in a physiological environment, it is predicted that only a small amount of natural immunoactive substance is obtained during the initial infection. Thus, from the viewpoint of a biological defense reaction, it is considered that it is extremely important to enhance or amplify the reaction of such a small amount of natural immunoactive substance. The aforementioned results strongly suggest that the membrane molecule (A) be such an amplifying factor.

### Example 13 Clarification of relationship between the present membrane molecule and type I interferon receptor

It has been known that expression of a type I interferon receptor is important for generation of type I IFN from PDC. This is because expression of IRF7 that is a transcription factor essential for generation of type I IFN is promoted by stimulation from the type I interferon receptor (IFNαβR), so that Positive Feedback Amplification Loop can be established. Thus, an increase in expression of the membrane molecule (A) was confirmed in an IFNαβR knockout mouse.

Specifically, expression of the membrane molecule (A) from mouse splenic cells was confirmed by the methods described in Examples 10 and 11 using a flow cytometer.

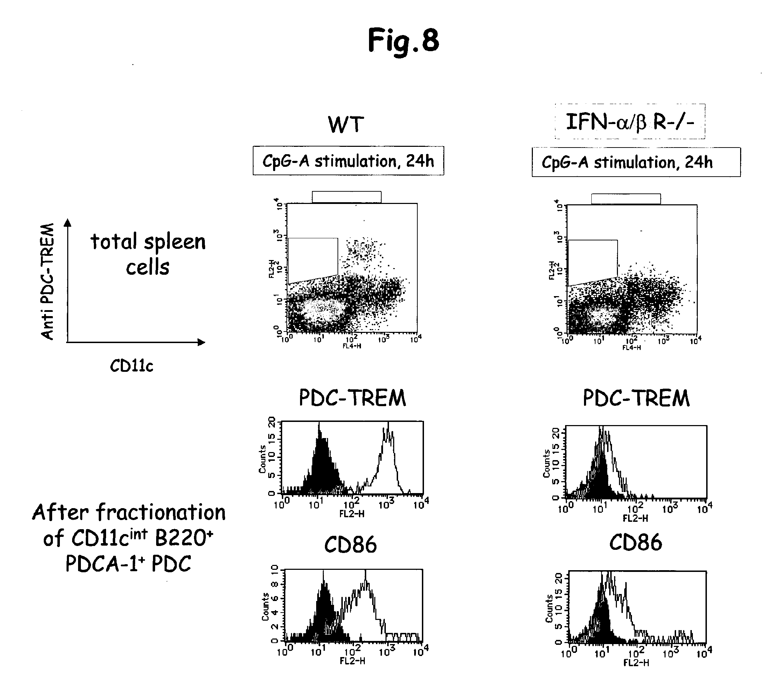

As a result, it became clear that expression of the membrane molecule (A) was not increased in such an IFNαβR knockout mouse (Figure 8). This result and the result of Example 12 demonstrated that the membrane molecule (A) was a molecule whose expression was increased with a Positive Feedback Amplification Loop. These results suggested that such an increase in the expression would be important for generation of type I IFN.

### Example 14 Identification of receptor complex of the present membrane molecule

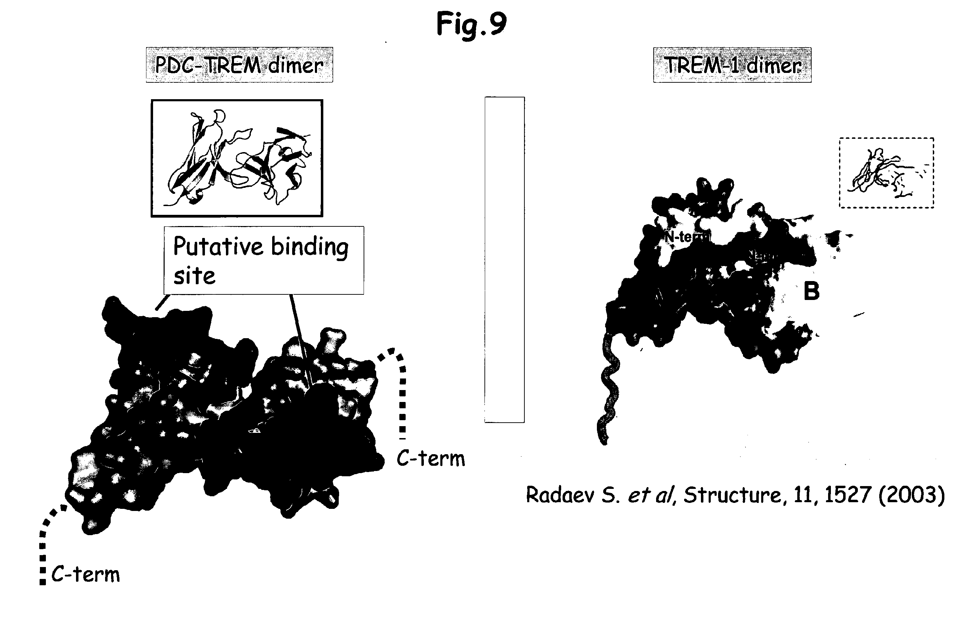

Based on the results of the crystal structure analysis of TREM-1 showing identity at an amino acid sequence level of 48% with the extracellular domain of the present membrane molecule (A), a steric structure was estimated by homology modeling (Figure 9). TREM-1 forms a unique head-to-tail dimer. Likewise, it was suggested that the membrane molecule (A) would be able to form a similar head-to-tail dimer. It has been reported that a ligand-binding site exists in a common loop portion (the red portion in Figure 9). Unexpectedly, it became clear that the estimated ligand-binding site between TREM-1 and the membrane molecule (A) is located on the lateral face of the molecule. This result strongly suggests that the membrane molecule (A) be associated with other molecules on the membrane.

### Example 15 Identification of molecule associated with the present membrane molecule

Based on the findings obtained in Example 14, an attempt was made to identify a molecule that was associated with the membrane molecule (A). After the cell membrane surface of bone marrow cell-derived PDC had been biotinylated, the cells were solubilized with a surfactant, followed by immunoprecipitation with a polyclonal antibody reacting with the membrane molecule (A). The sample was electrophoresed by SDS-PAGE, and it was then transferred onto a PVDF membrane. Thereafter, detection was carried out with HRP-conjugated avidin.

Specifically, bone marrow cell-derived plasmacytoid dendritic cells prepared by the method described in Example 12 were reacted with the biotinylating reagent described in Example 10 in PBS in ice for 1 hour, thereby biotinylating the cell surface. The bone marrow cell-derived plasmacytoid dendritic cells, the surface of which was biotinylated, were solublized in 1% NP-40-containing PBS. The antibody described in Example 8 was added thereto, followed by o/n reaction at 4°C. Thereafter, an antibody-antigen complex was recovered by immunoprecipitation using Protein G agarose. A sample buffer was added to the recovered sample, and it was then boiled at 95°C for 5 minutes. The resultant sample was used as a detection sample.

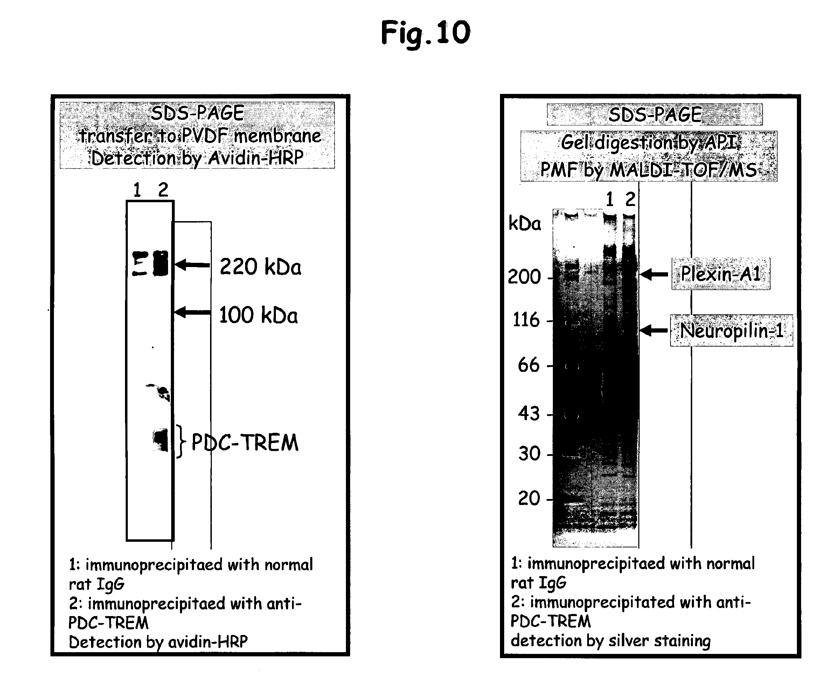

As a result, bands estimated to be molecules associated with the membrane molecule (A) were detected at 220 kDa and 100 kDa (left figure of Figure 10). Subsequently, membrane molecules were prepared by the methods described in Examples 1 and 3, and immunoprecipitation using a polyclonal antibody reacting with the membrane molecule (A) was performed thereon. After completion of electrophoresis by SPS-PAGE, proteins were detected by the method described in Example 2. As a result, candidate bands were detected in the same positions as those described above. The bands were cut out and were then digested with API. Thereafter, molecules were identified by the identification method described in Example 4. As a result, it was found that the band at 220 kDa was Plexin-A1 and that the band at 100 kDa was Neuropilin-1 (right figure of Figure 10).

### Example 16 Analysis of state of the present membrane molecule associated with Plexin-A1 and Neuropilin-1

Based on the descriptions of Example 15, the state of the present membrane molecule (A) that was associated with Plexin-1 and Neuropilin-1 was analyzed. The full-length gene of the present membrane molecule (A), and the full-length genes of DAP12, Plexin-A1 and Neuropilin-1, to which each different tag had been attached, were transfected in various combinations.

Specifically, the full-length genes of FLAG tag-attached DAP12, Myc tag-attached Plexin-A1, HA tag-attached Neuropilin-1, and the membrane molecule (A), were inserted into an expression vector. The fact that each gene is expressed on 293T cells by the method described in Example 9 was confirmed by the Western blotting method described in Example 12, using each specific antibody. Various combinations of expression vectors were co-transfected in 293T cells, and immunoprecipitation was then performed by the method described in Example 15 using an antibody reacting with the membrane molecule (A). Coprecipitation of the recovered samples was confirmed by the Western blotting method described in Example 12 using each specific antibody.

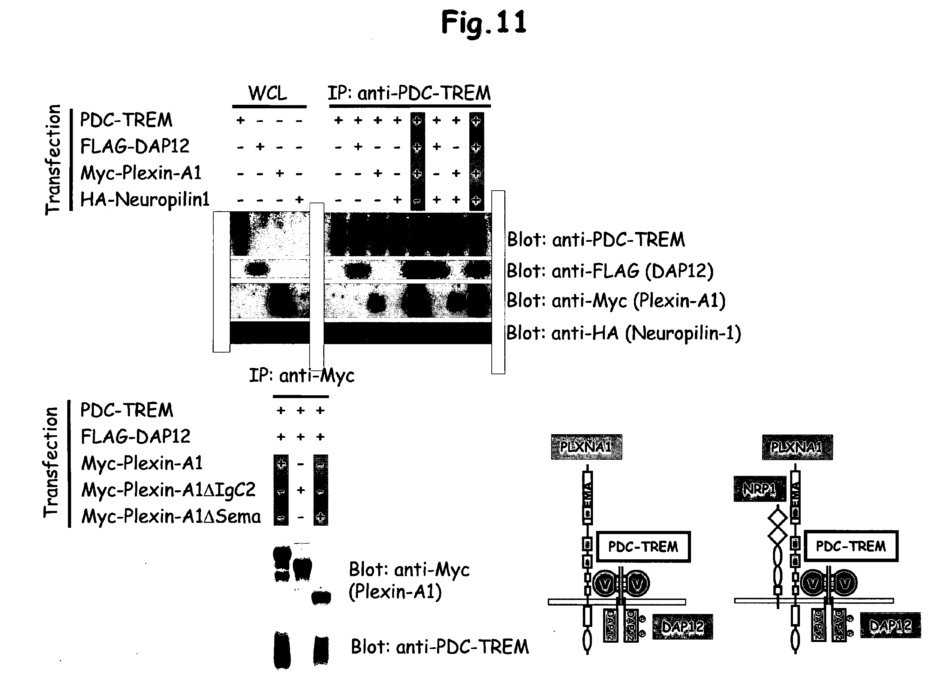

The results of immunoprecipitation with an antibody reacting with the membrane molecule (A) and detection of a tag sequence suggested that the membrane molecule (A) be associated with Plexin-A1 and that Neuropilin-1 be associated with Plexin-A1. These results suggested that the two types of receptor complexes as shown in Figure 11 could be present. Moreover, as a result of the analysis of a deletion mutant of Plexin-A1, it was suggested that the membrane molecule (A) be associated with an immunoglobulin domain directly on the cell membrane of Plexin-A1.

### Example 17 Expression of ligand to the present membrane molecule complex in immunocompetent cells

It has been reported that the ligand to single Plexin-A1 is a membrane-type Semaphorin, Sema6D, and that the ligand to a Plexin-A1/Neuropilin-1 complex is solubilized-type Semaphorin, Sema 3A. Expression of Sema6D and Sema3A in immunocompetent cells was confirmed by RT-PCR and DNA microarray.

The cells were stained with a cell surface marker specific for each type of cell, and as described in Example 11, the cells were separated using a cell sorter. Specific examples of such cells include CD3-positive T cells, CD19-positive B cells, NK1.1-positive CD3-negative NK cells, CD11c-positive CD11b-positive B220-negative conventional DC, and CD11c-positive CD11b negative B220-positive plasmacytoid DC. From such cells, total RNA was prepared using RNeasy kit (manufactured by QIAGEN). The obtained total RNA was used to confirm expression of a specific gene, using One step RT-PCR kit (manufactured by Invitrogen). In addition, after the synthesis of complementary RNA, it was used to confirm expression of the entire gene with a DNA microarray (manufactured by Affimetrix).

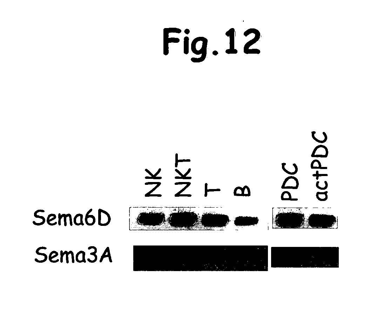

As a result, it became clear that expression of Sema3A was not observed, but that Sema6D was expressed not only in effector cells such as NK cells, NKT cells, T cells, or B cells, but also in activated epithelial cells or in PDC itself (Figure 12).

### Example 18 Change in function of PDC by stimulation with Sema6D

As described in Example 17, it became clear that the membrane molecule (A) forms a receptor complex, and that Sema6D as a ligand thereto is expressed on immunocompetent cells. Thus, the extracellular domain of Sema6D was allowed to express in the form of a recombinant form of an Ig fusion (SEQ ID NOS: 9 and 10; attached as a text file). Thereafter, spleen-derived PDC was cultured on a plate on which the recombinant form had been immobilized, in the presence of various concentrations of CpG-A.

The extracellular domain of Sema6D was fused with the Fc domain of human IgG1 (hereinafter referred to as Sema6DIg), and the fused product in a pFastBac vector (manufactured by Invitrogen) was allowed to express in Sf9 cells in a vaculovirus system. A recombinant form was prepared from a serum free culture supernatant by affinity purification using Protein A Sepharose (manufactured by Amersham Biosciences). 10 µg/ml Sema6DIg was immobilized on a 96-well plate, and PDC prepared by the method described in Example 11 was inoculated thereon in a concentration of 1 x 10⁵ cells. CpG-A was added thereto in concentrations of 0.01 µM, 0.1 µM, and 1 µM, so as to stimulate the PDC. Twenty-four hours after the culture, the concentration of IFNalpha was measured by ELISA (manufactured by PBL Biomedical Laboratories).

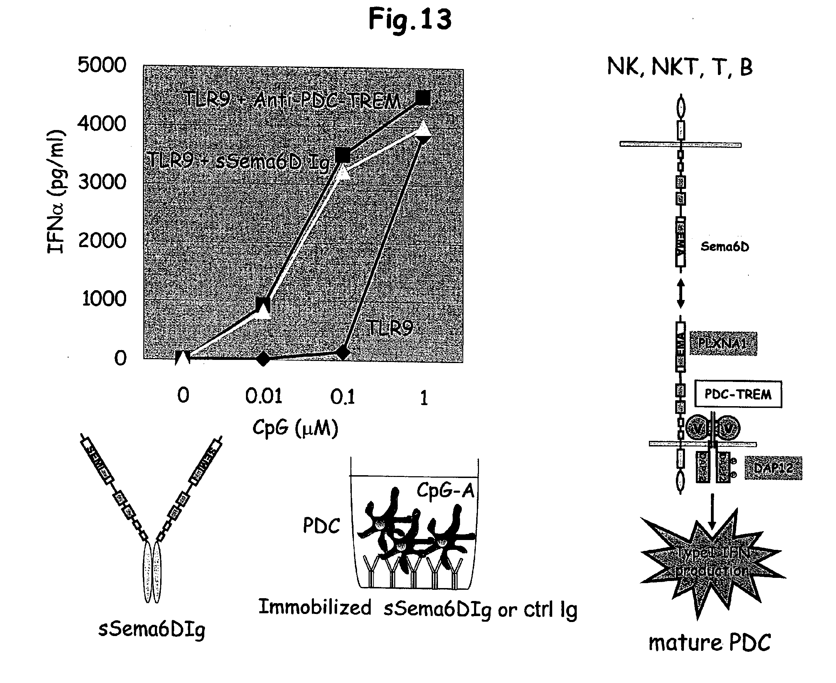

As a result, it became clear that generation of type I IFN was promoted by a signal from the membrane molecule (A) (Figure 13). In particular, by stimulation with a low concentration of CpG, the amount of type I IFN generated was 10 to 100 times increased. Considering virus infection in a physiological environment, it is predicted that only a small amount of natural immunoactive substance is obtained during the initial infection. Thus, from the viewpoint of a biological defense reaction, it is considered that it is extremely important to enhance or amplify the reaction of such a small amount of natural immunoactive substance. The aforementioned results strongly suggest that Sema6D contribute to such amplification as a ligand to the membrane molecule (A).

### Example 19 Analysis of expression of the present membrane molecule in vivo

As described in Examples 9 to 18, it became clear that the present membrane molecule (A) is expressed *in vitro* specifically for activated PDC and regulates generation of type I IFN. Thus, expression of the membrane molecule (A) *in vivo* was confirmed using a fluorescence microscope or a confocal laser microscope.

The cervical lymph node was obtained from a mouse, the footpad of which had been immunized with CpG-A (Figures 14 and 15), or the spleen was obtained from a mouse to which iv had been administered (Figures 16 and 17). The obtained organ was subjected to immunohistological staining. An anti-IgM antibody that specifically stains B cells, an anti-CD3 antibody that specifically stains T cells, an anti-PDCA-1 antibody that specifically stains PDC, and an anti-membrane molecule (A) antibody that specifically stains activated PDC, were used.

Specifically, the collected organ was mixed with Tissue Tek OCT (manufactured by Sakura), and it was then frozen at -80°C. The frozen tissues were sliced with a cryostat (manufactured by Leica Microsystems) so as to preapre tissue sections. Such tissue sections were stained with each of anti-CD3-biotin (T cell region; manufactured by BD Bioscience), anti-IgM-biotin (B cell region; manufactured by BD Bioscience), anti-PDCA-1-FITC (PDC; manufactured by Miltenyi Biotech), anti-membrane molecule (A)-biotin (activated PDC), and streptoavidin-Q-Dot605 (manufactured by QUANTUMDOT), so as to stain the aforementioned individual regions. The thus stained images were observed with a fluorescence microscope (manufactured by Nikon Corp.) or a confocal laser microscope (manufactured by BioRad).

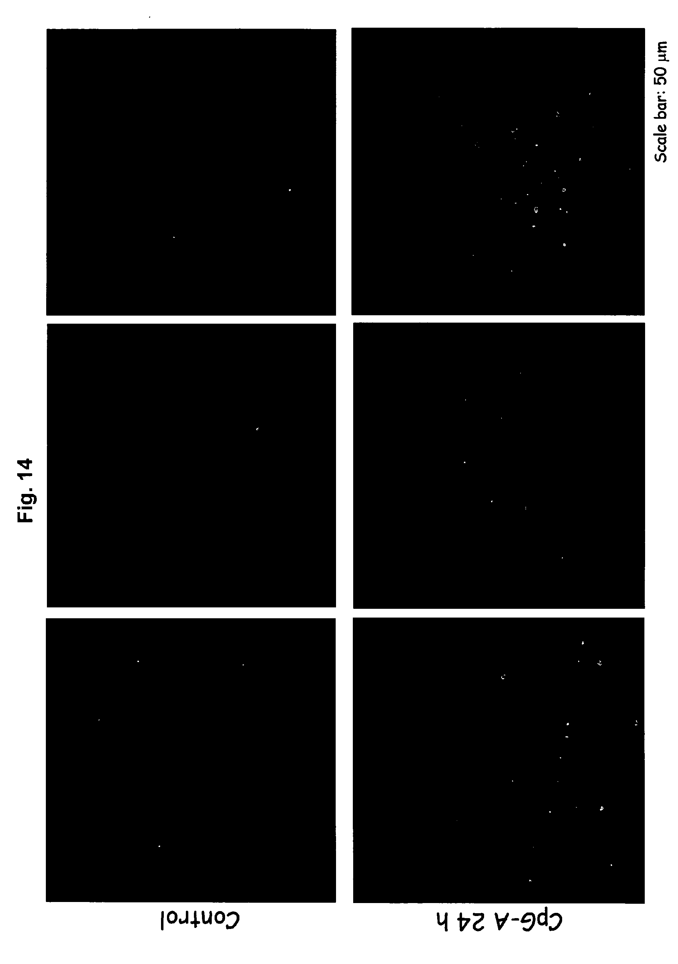

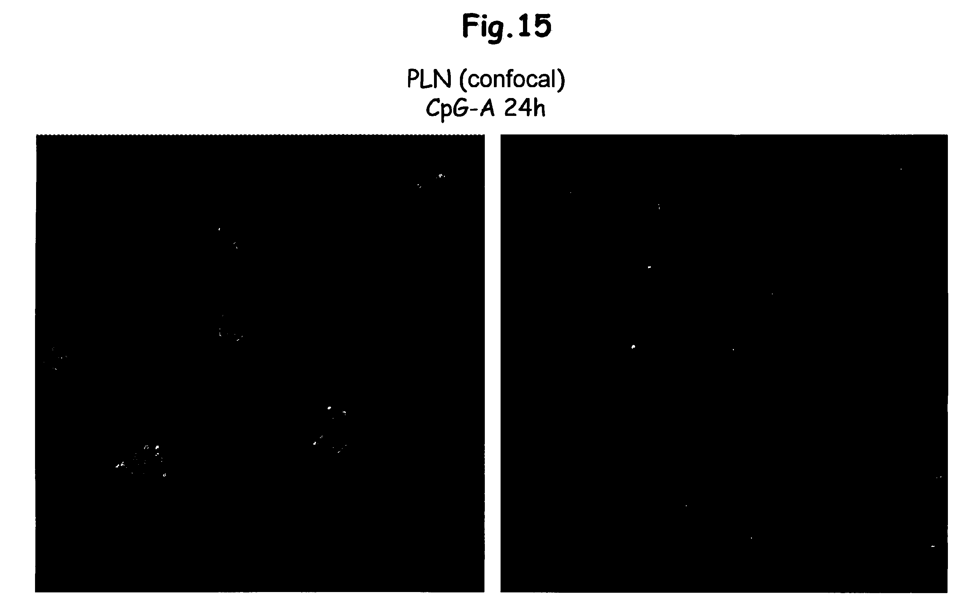

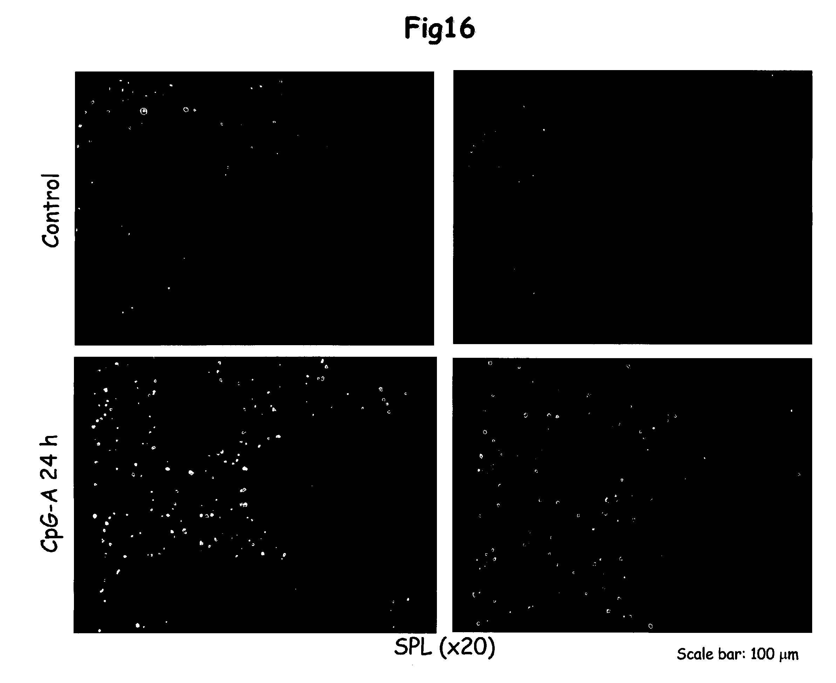

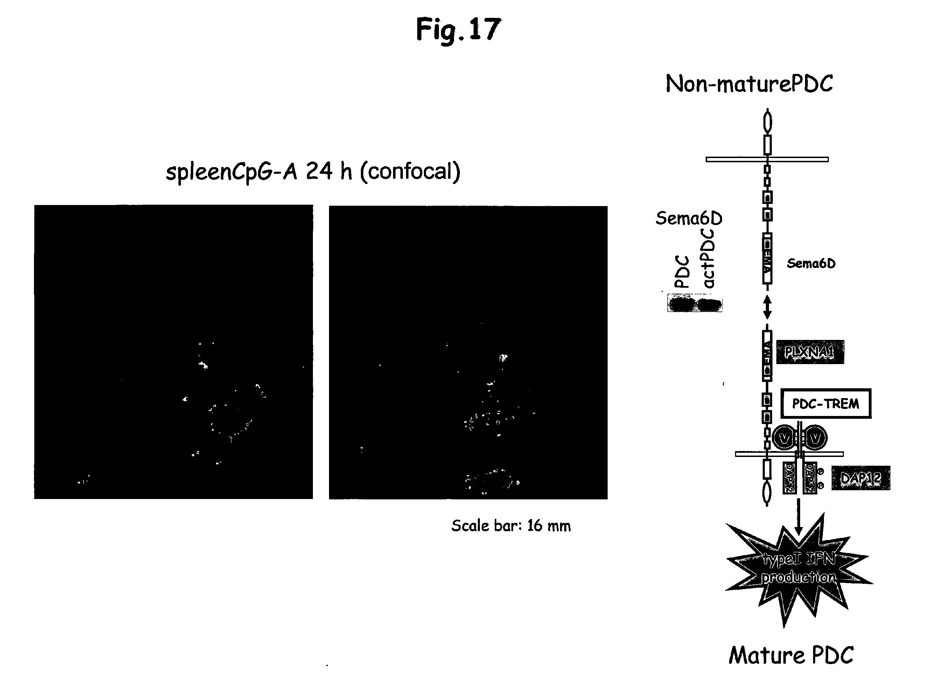

As shown in Figure 14, it was confirmed that PDC migrated to the regional lymph node as a result of administration of CpG-A, and at the same time it was activated, and that it infiltrated into the T cell region. As shown in Figure 15, the interaction between T cells and the activated PDC was also confirmed. In addition, as shown in Figure 16, it was confirmed that PDC formed a cluster in the spleen. As shown in Figure 17, with regard to such a PDC cluster, it was confirmed that the activated PDC was surrounded with deactivated PDC. As shown in Figure 17, since Sema6D was expressed on PDC itself, it was considered that this PDC cluster contributes to efficient generation of type I IFN.

### Example 20 Analysis of association of PDC-TREM with DAP12

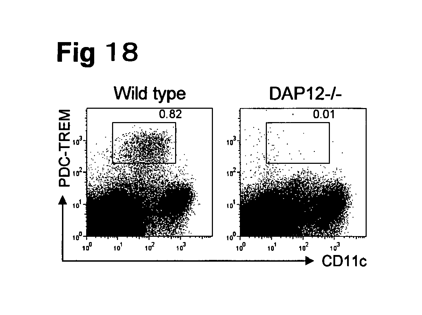

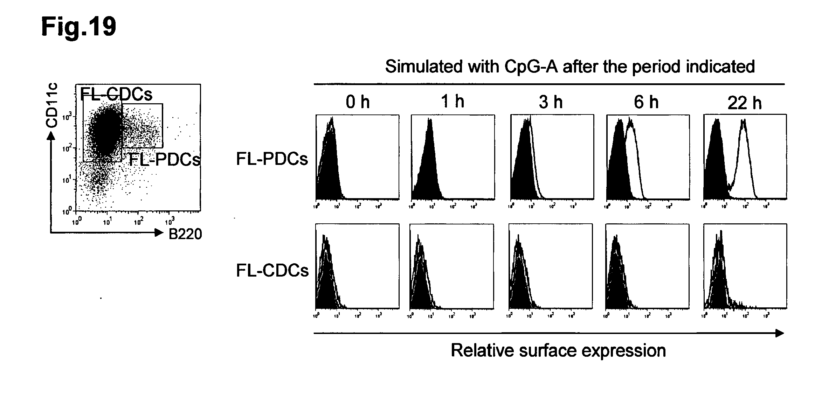

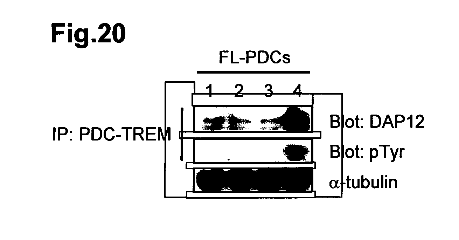

In order to confirm the association of PDC-TREM with DAP12 described in Example 9, expression of PDC-TREM in DAP12 knockout mouse splenic cells was confirmed by the same method as that described in Example 10. As a result, no expression of PDC-TREM was confirmed in the activated splenic cells. Thus, it was predicted that PDC-TREM was associated with DAP12 (Figure 18). Moreover, it was also revealed that an increase in expression of PDC-TREM as a result of stimulation with CpG-A was observed in PDC induced by culturing mouse bone marrow in the presence of Flt3 ligand for 10 days (FL-PDC) (Figure 19). Furthermore, it was confirmed that DAP12 associated with PDC-TREM in CpG-A-stimulated FL-PDC was phosphorylated (Figure 20). These results strongly suggest that PDC-TREM be associated with DAP 12, and that DAP12 be an adaptor involved in signaling.

### Example 21 Search for factor causing increase in expression of PDC-TREM

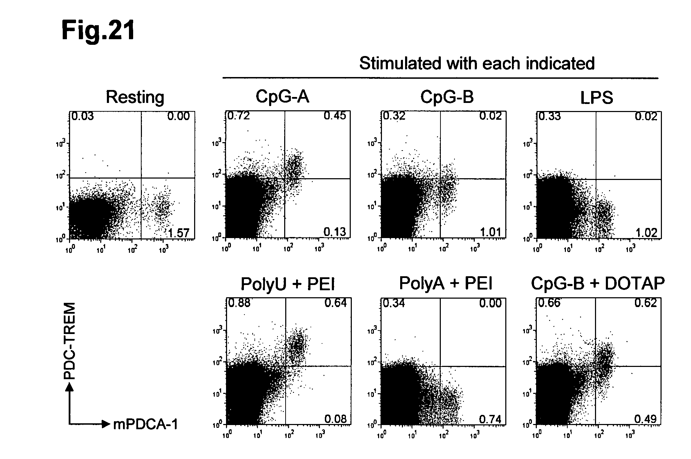

As described in Example 11, an increase in expression of PDC-TREM in PDC as a resutl of stimulation with CpG-A could be confirmed. An increase in expression of PDC-TREM in PDC by various types of other stimulations was analyzed (Figure 21). As a result, such an increase in expression of PDC-TREM in PDC was observed in the case of PolyU introduced using PEI or in the case of CpG-B introduced using DOTAP, but such an increase in expression was not observed in the case of CpG-B or in the case of PolyA introduced using high-purity LPS or PEI. These results have a correlation with an increase in expression of PDC-TREM in type I IFN-generating PDC. Thus, these results strongly suggest that PDC-TREM be involved in the mechanism of PDC for generating type I IFN.

### Example 22 Change in funciton of PDC caused by allowing blocking antibody to act on PDC-TREM

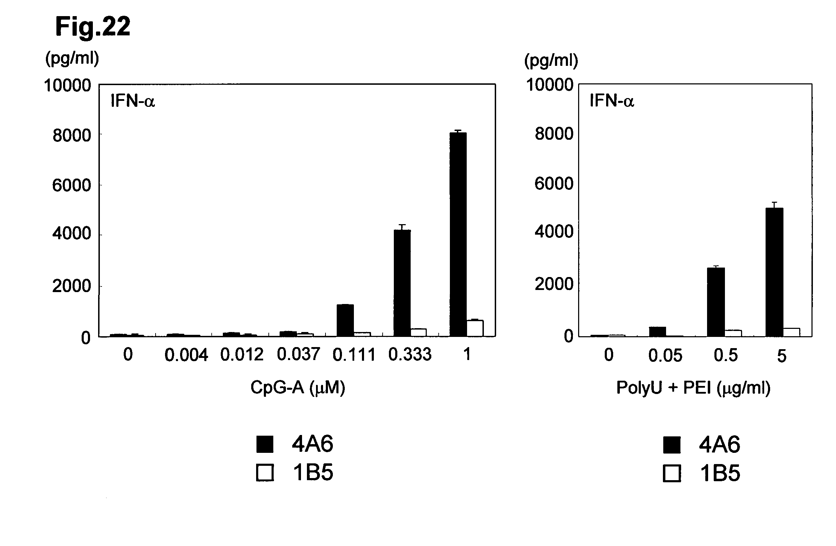

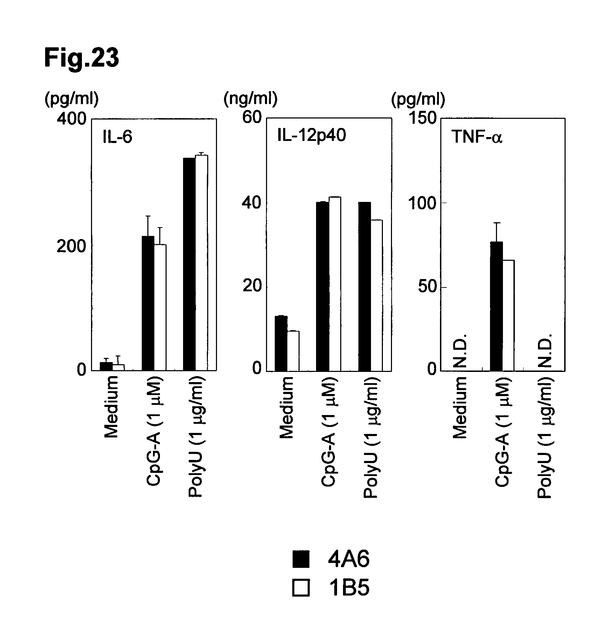

Using a monoclonal antibody specific for PDC-TREM, the influence of the aforementioned antibody on the ability of CpG-A- or PolyU-stimulated FL-PDC to generate type I IFN and the generation of inflammatory cytokines was analyzed. As a result, it became clear that a monoclonal antibody 1B5 suppressed generation of type I IFN from FL-PDC to a level of approximately 1/10 to 1/100 (Figure 22). On the other hand, with regard to inflammatory cytokines such as TNFalpha, IL-6 or IL-12, generation ability was not changed by the treatement with the aforementioned antibody (Figure 23). These results strongly suggest that PDC-TREM be a molecule which is specifically involved in the control of generation of type I IFN in PDC. In addition, another specific monoclonal antibody 4A6, which has no influence on the ability of CpG-A- or PolyU-stimulated FL-PDC to generate type I IFN and the generation of inflammatory cytokines, was also established.

### Example 23 Clarification of signaling pathway downstream of PDC-TREM

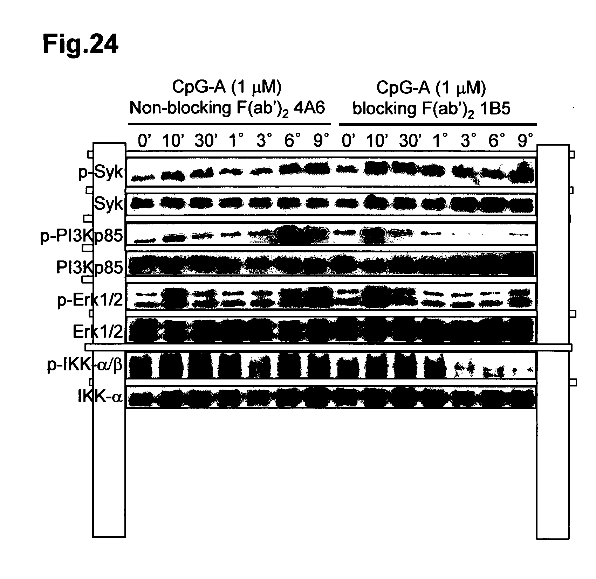

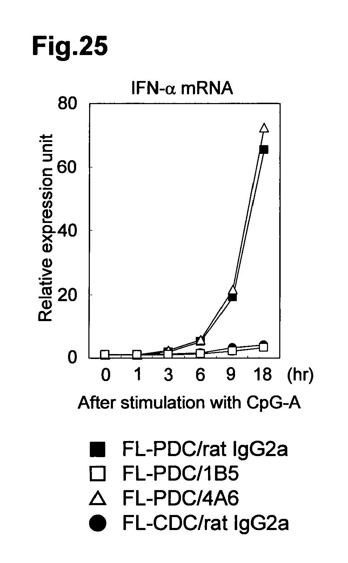

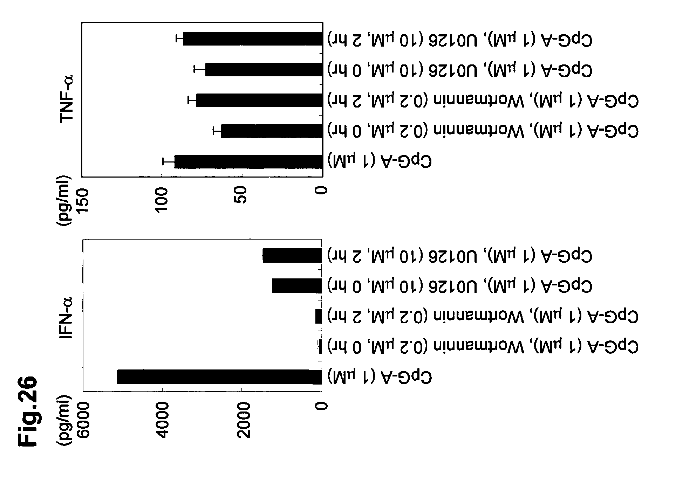

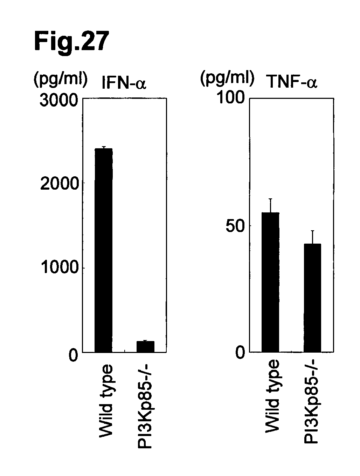

As described in Examples 9, 10, 20 and 22, it is considered that PDC-TREM is associated with DAP12. Based on this assumption, whether or not a molecular group presumably associated with signaling downstream of DAP12 is involved in generation of type I IFN in PDC was confirmed using phosphorylation of each molecule as an indicator. As shown in Figure 22, since 1B5 specifically suppressed generation of type I IFN, phosphorylation of Syk, PI3K and Erk1/2 in 1B5- or 4A6-treated CpG-A-stimulated FL-PDC was observed over time. As a result, it was found that phosphorylation of such molecules was suppressed 3 to 9 hours after stimulation iwht CpG-A (Figure 24). Moreover, since suppression of phosphorylation had a temporal correlation with the expression level of mRNA of IFNalpha (Figure 25), it was strongly suggested that a signaling pathway downstream of PDC-TREM be involved in generation of type I IFN. Furthermore, it was also confirmed that phosphorylation of IKKalpha, a signaling molecule known to be involved in an increase in genreation of type I IFN and to bind to IRF7, was also suppressed with 1B5 (Figure 24). Further, almost no IFNalpha was generated as a result of stimulation with CpG-A in FL-PDC with Wortmannin or U0126 that were specific inhibitors of PI3K or MEK1/2 (located directly above Erk1/2) (Figure 26). Still further, almost no IFNalpha was generated as a result of stimulation with CpG-A in FL-PDC induced from the bone marrow cells of a PI3Kp85 knockout mouse (Figure 27). These results suggest that PDC-TREM and a signaling pathway downstream thereof play an important role in generation of type I IFN in PDC.

### Example 24 Clarification of signaling pathway by stimulation with Sema6D

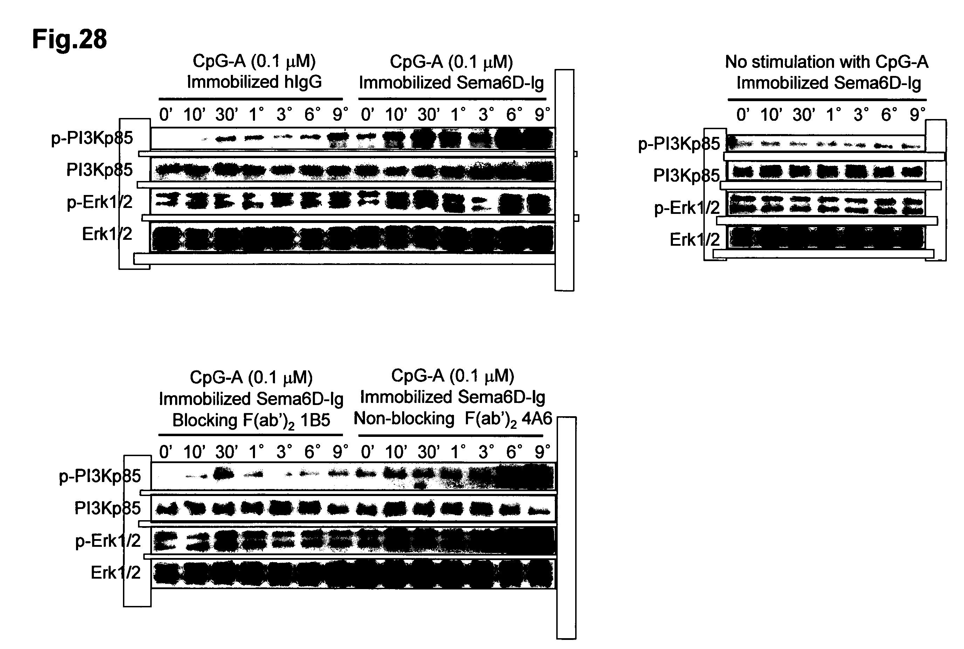

As described in Examples 17 and 18, it became clear that PDC-TREM formed a receptor complex with PlexinA1, and that Sema6D considered as a ligand of PDC-TREM/PlexinA1 promoted generation of type I IFN from PDC. Thus, phosphorylation of PI3K and Erk1/2 by stimulation with Sema6D was observed over time. As a result, it was confirmed that phosphorylation of PI3K and Erk1/2, regarding which phosphorylation was suppressed in Examle 23, was accentuated for 3 to 9 hours, and that Sema6D-dependent phosphorylation was suppressed with 1B5 but it was not suppressed with 4A6 (Figure 28). These results strongly suggest that Sema6D may phosphorylate, PDC-TREM-dependently, signaling molecules such as PI3K or Erk1/2 and contribute to generation of type I IFN.

### Example 25 Clarification of role of PDC-TREM in biological defense mechanism during virus infection

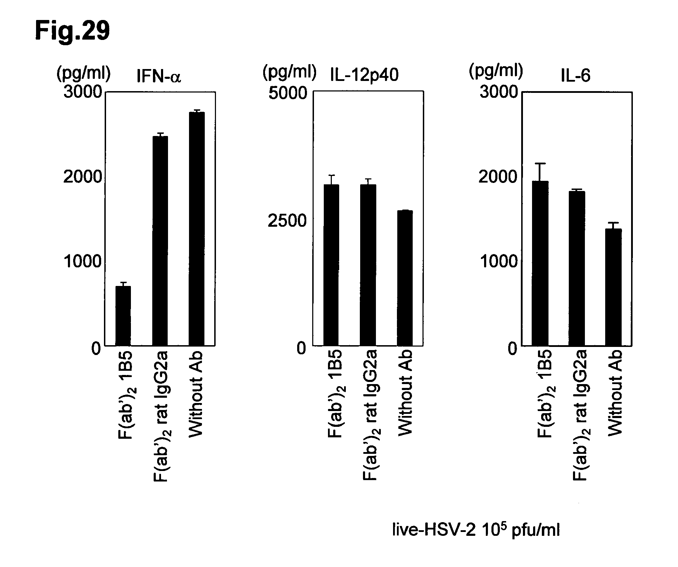

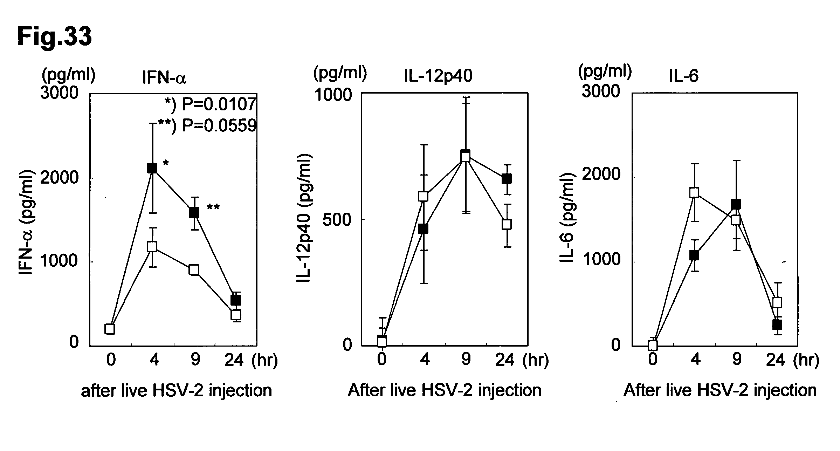

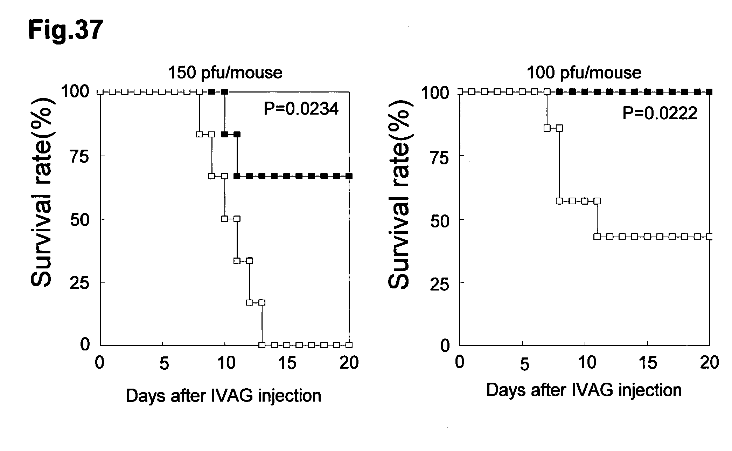

It has been known that PDC expresses a receptor recognizing varous types of virus-derived nucleic acids, such as TLR9 or TLR7, and expresses a large amount of type I IFN as a result of ligand recognition, and thus that PDC plays a main role in an anti-viral biological defense mechanism. Based on the fact that PDC-TREM is expressed at a high level in PDC after TLR stimulation and that it is involved in generation of type I IFN (Examples 13, 18, 22 and 24), whether or not PDC-TREM is involved in a biological defense mechanism against viruses was confirmed using herpes simplex virus (HSV) as DNA virus and also using vesicular stomatitis virus (VSV) as RNA virus. As a result, it became apparent that IFNalpha generated by stimulating FL-PDC with HSV or VSV *in vitro* was suppressed at a level of 80% or more by 1B5 in both live virus and UV-inactivated virus (Figures 29, 30, 31 and 32). On the other hand, almost no change was observed in generation of inflammatory cytokines such as IL-12 or IL-6 (Figures 29, 30, 31 and 32). Thus, whether or not PDC-TREM is involved in generation of IFNalpha even *in vivo* was analyzed. After intraperitoneal administration of 1B5, the concentration of cytokine in the blood of a mouse infected with virus via intravenous injection was measured. As a result, it became apparent that, as in the case of generation of IFNalpha *in vitro,* IFNalpha generated by stimulating FL-PDC with HSV or VSV *in vivo* was suppressed at a level of 80% or more by 1B5 in both live virus and UV-inactivated virus (Figures 33, 34, 35 and 36). On the other hand, almost no change was observed in generation of inflammatory cytokines such as IL-12 or IL-6 (Figures 33, 34, 35 and 36). Thereafter, resistance to virus infection was examined based on a survival rate. Survival resistance to HSV-2 that had been transvaginally infected into mice on a basis of infected human models was analyzed using 1B5. As a result, it was confirmed that 1B5-administered mice died at a high rate (Figure 37). Accordingly, it became apparent that generation of type I IFN from PDC by PDC-TREM is important for a defense mechanism to viruses.

### INDUSTRIAL APPLICABILITY

The present invention enables selective separation of activated (mature) PDC from other types of blood cells at high purity, and also enables separation of mature DC from immature DC. Thus, the present invention enables application of the aforementioned cells to DC therapy. An antigen is pulsed to the separated DC, and the DC is then returned to a patient again. Thus, it is anticipated that the DC will be used as a cancer vaccine. In addition, PDC has also been known as a strong type I interferon-producing cell (IPC), and it is anticipated that PDC will be applied to the treatment of autoimmune disease such as SLE, the symptoms of which are deteriorated due to an increase in generation of type I interferon in blood. Moreover, it is also anticipated that an antibody reacting with the present membrane molecule or the solubilized molecule of the present membrane molecule will be used to inhibit the interaction between DC and T cells, so as to control immune responses.

### BRIEF DESCRIPTION OF THE DRAWINGS

Figure 1 shows a hydropathy plot analysis performed on the present membrane molecule by the method of Kyte and Doolittle.
Figure 2 shows expression and purification of a recombinant form. DTT+ (monomer 0): 60 kDa, DTT- (dimer): 120 kDa. Protein A sepharose was used in purification, and silver staining was carried out (5/20 gradient gel, 1: input, 2: flow-through, 3: eluent).
Figure 3 shows the analysis of a cell population in which the membrane molecule (A) has been expressed, using a flow cytometer.
Figure 4 shows a search for an adaptor molecule associated with the membrane molecule (A).
Figure 5 shows a detailed analysis of a membrane molecule (A)-positive cell population, using a flow cytometer.
Figure 6 shows an increase in expression of PDC-TREM by CpG-A. (Hereinafter, the term "PDC-TREM" used in the figures means the membrane molecule (A) of the present invention.)
Figure 7 shows that an agonistic antibody activates PDC-TREM, so that type I IFN is dose-dependently generated from PDC.
Figure 8 shows that PDC-TREM is not expressed on the CpG-A-stimulated PDC of an IFNabR-deficient mouse.
Figure 9 shows an estimated structural model of PDC-TREM. When compared with a head-to-head dimer, a PDC-TREM head-to-head dimer requires a receptor to take a different direction regarding membrane.
Figure 10 shows identification of an associated molecule that is associated with the membrane molecule (A).
   (Left figure) Separation by SDS-PAGE, transcription onto a PVDF membrane, detection with avidin-HRP; 1: immunoprecipitation with normal rat IgG; 2: immunoprecipitation with anti-PDC-TREM; detection with avidin-HRP
   (Right figure) Separation by SDS-PAGE, gel digestion with API, PMF with MALDI-TOF/MS; 1: immunoprecipitation with normal rat IgG; 2: immunoprecipitation with anti-PDC-TREM, and detection by silver staining.
Figure 11 shows the analysis of a membrane molecule (A) /receptor complex.
Figure 12 shows the analysis of expression of Sema 6D and Sema 3A in immunocompetent cells.
Figure 13 shows that Sema 6D acts on PDC to promote generation of type I IFN.
Figure 14 shows transition of mature PDC to LN after administration of CpG-A.
Figure 15 shows a direction interaction between mature PDC and T/NKT cells in LN after administration of CpG-A.
Figure 16 shows localization of a PDC cluster in splenic red pulp after administration of CpG-A.
Figure 17 shows localization of mature PDC in the center of the cluster.
Figure 18 shows expression of PDC-TREM in splenic cells stimulated by CpG-A derived from a WT or DAP 12 knockout mouse.
Figure 19 shows expression of PDC-TREM stimulated by CpG-A in FL-CDC or FL-PDC.
Figure 20 shows that DAP12 is associated with PCD-TREM in CpG-A-stimulated FL-DPC, and it is phosphorylated.
Figure 21 shows expression of PDC-TREM in PDC in splenic cells after various types of stimulations.
Figure 22 shows generation of IFNalpha in 1B5- or 4A6-treated CpG-A-stimulated or PolyU-stimulated FL-PDC.
Figure 23 shows generation of inflammatory cytokine in 1B5- or 4A6-treated CpG-A-stimulated FL-PDC.
Figure 24 shows phosphorylation of a signaling molecule in 1B5- or 4A6-treated CpG-A-stimulated FL-PDC.
Figure 25 shows the relative RNA amount of IFNalpha4 in 1B5- or 4A6-treated CpG-A-stimulated FL-PDC or FL-PDC.
Figure 26 shows generation of IFNalpha in CpG-A-stimulated FL-PDC in the presence of a PI3K or Mek1/2 inhibitor.
Figure 27 shows generation of IFNalpha in CpG-A-stimulated FL-PDC that was derived from a WT or PI3Kp85 knockout mouse.
Figure 28 shows generation of IFNalpha in CpG-A-stimulated FL-PDC in the presence of Sema6D.
Figure 29 shows generation of IFNalpha and inflammatory cytokine in 1B5- or 4A6-treated live HSV-stimulated FL-PDC.

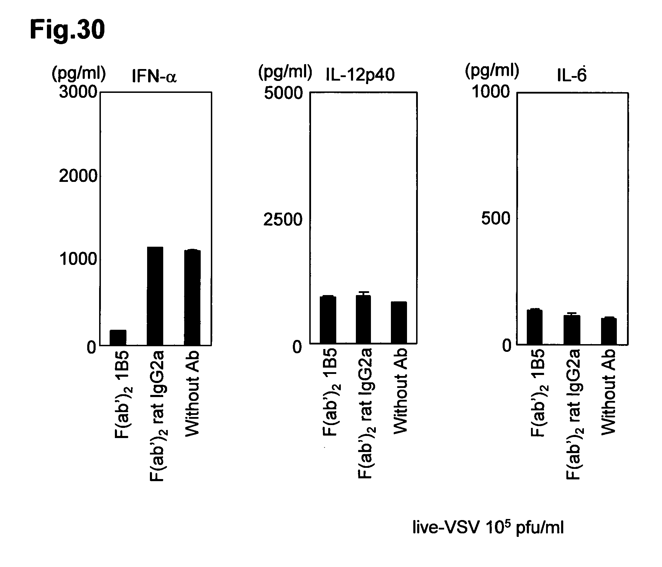

Figure 30 shows generation of IFNalpha and inflammatory cytokine in 1B5- or 4A6-treated live VSV-stimulated FL-PDC.

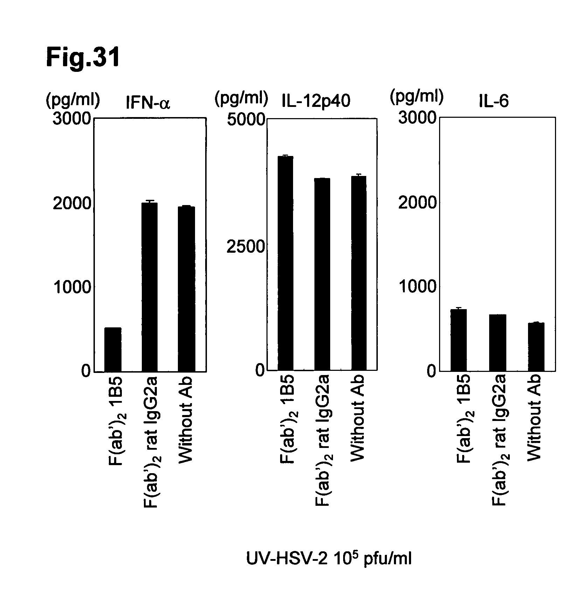

Figure 31 shows generation of IFNalpha and inflammatory cytokine in 1B5- or 4A6-treated UV-inactivated HSV-stimulated FL-PDC.

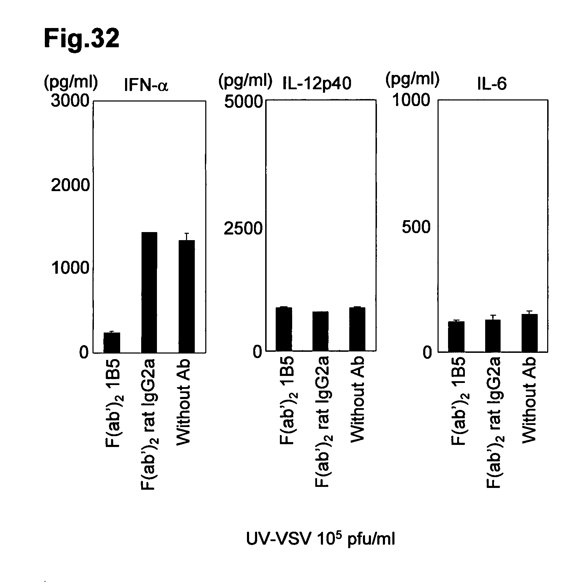

Figure 32 shows generation of IFNalpha and inflammatory cytokine in 1B5- or 4A6-treated UV-inactivated VSV-stimulated FL-PDC.
Figure 33 shows the concentration of IFNalpha and inflammatory cytokine in the blood of a mouse, to which 1B5 or rat IgG2a was administered, and which was then infected with live HSV.

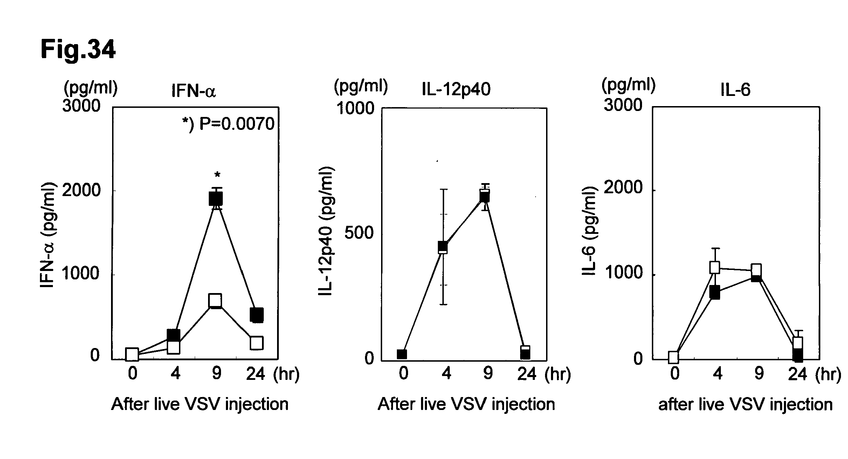

Figure 34 shows the concentration of IFNalpha and inflammatory cytokine in the blood of a mouse, to which 1B5 or rat IgG2a was administered, and which was then infected with live VSV.

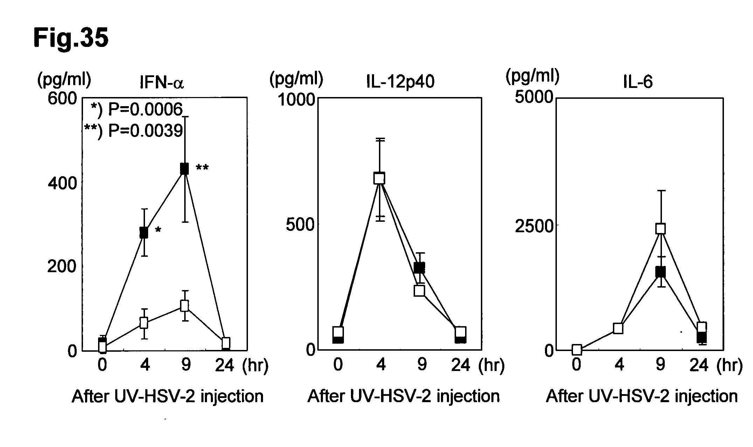

Figure 35 shows the concentration of IFNalpha and inflammatory cytokine in the blood of a mouse, to which 1B5 or rat IgG2a was administered, and which was then infected with UV-inactivated HSV

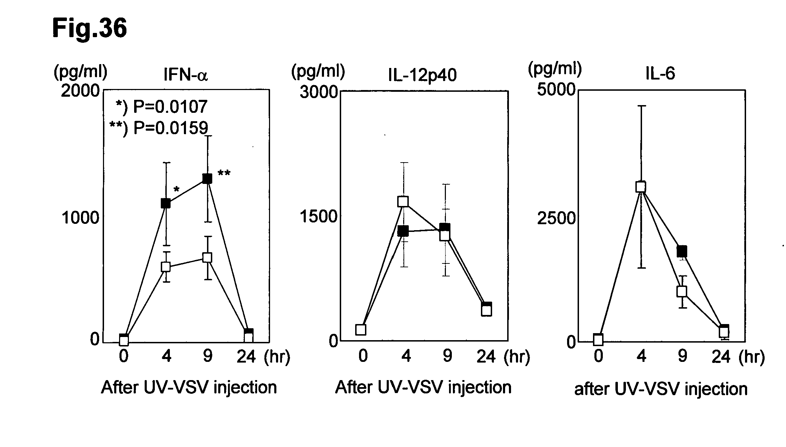

Figure 36 shows the concentration of IFNalpha and inflammatory cytokine in the blood of a mouse, to which 1B5 or rat IgG2a was administered, and which was then infected with UV-inactivated VSV.
Figure 37 shows the survival rate of a mouse, to which 1B5 or rat IgG2a was administered, and which was then infected with live HSV.

## Claims

1. A protein having any one of the following amino acid sequences:
(a) an amino acid sequence as shown in SEQ ID NO: 2;
(b) an amino acid sequence, which comprises a deletion, substitution, insertion and/or addition of one or several amino acid residues with respect to the amino acid sequence as shown in SEQ ID NO: 2, and which is capable of regulating the functions of dendritic cells; and
(c) an amino acid sequence, which has homology of 80% or more with the amino acid sequence as shown in SEQ ID NO: 2, and which is capable of regulating the functions of dendritic cells.

2. A regulator of the functions of dendritic cells, which comprises the protein of claim 1.

3. The regulator of the functions of dendritic cells of claim 2, which is used to increase generation of type I interferon.

4. DNA encoding the protein of claim 1.

5. DNA having any one of the following nucleotide sequences:
(a) a nucleotide sequence as shown in SEQ ID NO: 1;
(b) a nucleotide sequence, which comprises a deletion, substitution, insertion and/or addition of one or several nucleotides with respect to the nucleotide sequence as shown in SEQ ID NO: 1, and which encodes an amino acid sequence capable of regulating the functions of dendritic cells; and
(c) a nucleotide sequence, which hybridizes with the nucleotide sequence as shown in SEQ ID NO: 1 or a complementary sequence thereof under stringent conditions, and which encodes an amino acid sequence capable of regulating the functions of dendritic cells.

6. A recombinant vector, which comprises the DNA of claim 4 or 5.

7. A transformant, which comprises the DNA of claim 4 or 5 or the recombinant vector of claim 6.

8. An antibody, which specifically immunologically binds to the protein of claim 1 or a fragment thereof.

9. The antibody of claim 8, which immunologically recognizes plasmacytoid dendritic cells.

10. The antibody of claim 8, which recognizes the extracellular region of the protein of claim 1.

11. The antibody of any one of claims 8 to 10, which is either a polyclonal antibody or a monoclonal antibody.

12. A method for separating human- or other animals-derived plasmacytoid dendritic cells by using the antibody of any of claims 8 to 11.

13. A method for detecting plasmacytoid dendritic cells by using the antibody of any of claims 8 to 11.
